(19) Europäisches Patentamt
European Patent Office
Office européen des brevets

(11) EP 0 536 304 B1

(12) **EUROPEAN PATENT SPECIFICATION**

(45) Date of publication and mention
of the grant of the patent:
**04.12.1996 Bulletin 1996/49**

(51) Int Cl.[6]: **G01N 21/35**, A61B 5/00,
G01N 21/31

(21) Application number: **91913253.0**

(22) Date of filing: **27.06.1991**

(86) International application number:
**PCT/US91/04616**

(87) International publication number:
**WO 92/00513 (09.01.1992 Gazette 1992/02)**

(54) **NON-INVASIVE MEASUREMENT OF BLOOD GLUCOSE**

EINGRIFFSFREIE MESSUNG DER BLUTGLUKOSE

MESURE NON INVASIVE DE GLYCEMIE

(84) Designated Contracting States:
**AT BE CH DE DK ES FR GB GR IT LI LU NL SE**

(30) Priority: **27.06.1990 US 544580**
**10.08.1990 US 565302**
**09.04.1991 US 682249**

(43) Date of publication of application:
**14.04.1993 Bulletin 1993/15**

(73) Proprietor: **FUTREX, INC.**
**Gaithersburg, MD 20879 (US)**

(72) Inventors:
• **ROSENTHAL, Robert, D.**
**Gaithersburg, MD 21227 (US)**

• **PAYNTER, Lynn, H.**
**Elkridge, MD 21227 (US)**
• **MACKIE, Linda, H.**
**Rockville, MD 20853 (US)**

(74) Representative: **Pendlebury, Anthony et al**
**PAGE, WHITE & FARRER**
**54 Doughty Street**
**London WC1N 2LS (GB)**

(56) References cited:
**WO-A-90/07905**        **WO-A-91/11136**
**US-A- 4 805 623**       **US-A- 4 882 492**
**US-A- 4 883 953**

## Description

This invention relates to instruments and methods for the non-invasive quantitative measurement of blood analytes such as blood glucose.

Information concerning the chemical composition of blood is widely used to assess the health characteristics of both people and animals. For example, analysis of the glucose content of blood provides an indication of the current status of metabolism. Blood analysis, by the detection of above or below normal levels of various substances, also provides a direct indication of the presence of certain types of diseases and dysfunctions.

The normal method of determining blood chemistry is by removing a sample of blood (e.g. 5-10 ml) and performing one or more standard chemical tests. These types of tests are moderately expensive, require one class of trained technicians to remove the blood and another class of trained technicians to perform the chemical tests. Moreover, the results of the blood tests often are not available for several hours, and sometimes even several days.

Recently, an alternative type of technology (i.e. self-contained instruments) has been introduced for relatively rapid blood screening of a large number of subjects. These instruments, in general, use a much smaller blood sample (approximately .25 ml) from a "finger poke." This small blood sample is placed on a chemically-treated carrier and entered into the instrument. These instruments normally provide either an individual analysis (e.g., glucose level) or multiple analyses in a few moments. These types of instruments unfortunately are quite costly, e.g., in the range of several thousand dollars.

A third class of blood instrumentation is available for the specific purpose of determining glucose level in people with diabetes. This technology also uses a small sample from a finger poke and the sample is placed on a chemically treated carrier which is inserted into a portable battery operated instrument. In general, these instruments provide a single function, i.e., measurement of glucose. Although these specialized instruments are relatively low cost ($300 or less is typical), the cost of the disposable carrier "stick" must be considered. Since some diabetic patients may require glucose analysis four or more times a day, the cost over a period of a year can become significant.

Current glucose analytical systems require blood to be extracted from the body prior to performing the analysis. This blood withdrawal requirement limits the application of such testing; many people who may be interested in knowing their glucose level are reluctant to have either their finger poked or blood samples removed by hypodermic needle. This reluctance or anxiety in allowing blood sample removal is due to concern over the possibility of infection, discomfort (pain) and generalized patient fear.

Thus, there is a great need for non-invasive analytical instruments and methods that would provide essentially the same accuracy as conventional blood glucose tests. Moreover, there is a need for a non-invasive low-cost method for measurement of glucose in diabetic patients.

Near-infrared (sometimes referred to herein as simply "near-IR") quantitative analysis is widely used in the field of agriculture for determining chemical compositions within grain, oilseeds, and other agricultural products. As an example, near-IR energy reflected from the surface of finely ground seeds and grain provides information concerning protein and moisture content. For a general introduction to near infrared quantitative analysis, see "An Introduction to Near-Infrared Quantitative Analysis" presented by Robert D. Rosenthal at the 1977 Annual Meeting of the American Association of Cereal Chemists. Near-infrared technology has been extended to allow totally non-destructive measurements by using light transmission through a sample as discussed in "Characteristics of Non-Destructive Near-Infrared Instruments for Grain and Food Products" by Robert D. Rosenthal, presented at the 1986 Meeting at the Japan Food Science Institute. Although this transmission approach avoids the need to finely grind the sample, it is not suited for use where access to two opposite surfaces is not available.

One example of this transmission approach is provided in US-A-4,621,643 (New, Jr. et al., 1986) which relates to an optical oximeter apparatus for determining pulse rate and degree of arterial oxygen saturation. Light energy is passed through an appendage of the body, e.g. a finger, and strikes a detector positioned on a side of the appendage opposite from the light source. Pulse rate and saturated oxygen are calculated from coefficients of extinction of light at the selected wavelengths.

Another approach to near-infrared quantitative analysis, using near-infrared interactance, was developed for non-invasively measuring body fat content. This approach is described in "A New Approach for the Estimation of Body Composition: Infrared Interactance," Joan M. Conway et al., The American Journal of Clinical Nutrition, 40: Dec. 1984, pages 1123-1230. In this non-invasive technique, a small optical probe that allows optical energy to enter the arm is placed on the biceps. The percent body fat of the entire body is determined by measuring the spectrum change of the energy returned from an area adjacent the light entry point.

A limitation of the near-infrared blood glucose measurement instruments is that each may be required to be custom calibrated for each individual user. The need for individual custom calibration results from the different combination of water level, fat level and protein level in various individuals which causes variations in energy absorption. Since the amount of glucose in the body is less than one-thousandth of these other constituents, variations of these constituents which exist between people may make a universal calibration unlikely.

The current approach for custom calibrating near-infrared blood glucose measurement instruments is to use an in vitro technique that requires removing blood from the subject and having an automatic instrument measure the glucose level of that blood. Such in vitro measurements are typically made with either the commercially available Biostator or the experimental Kowarski Continuous Monitor. Each of the above instruments requires a catheter to be inserted into the subject and blood withdrawn over a one-to two-hour period. Although such an approach is feasible, it places a significant new burden on the docotor and the medical facility to have enough time, room and equipment to be able to calibrate instruments in this fashion.

Thus, there is a great need for a technique which allows an individual user to easily and reliably custom calibrate the near-infrared instrument.

US-A-4805623 discloses a near-infrared quantitative analysis instrument and a non-invasive method for quantitative analysis having the features of the pre-characterising portion of claims 1 and 5 respectively.

US-A-4882492, US-A-4883953 and WO-A-91/11136 and WO-A-90/07905 (both of which comprise part of the state of the art under Articles 54(3) and (4) EPC), each disclose an instrument and a method for near-infrared quantitative analysis of blood glucose.

In accordance with the present invention there is provided a near-infrared quantitative analysis instrument (10) for non-invasive measurement of blood glucose in blood present in a body part of a subject, comprising:

(a) introducing means including a near infrared energy source (16,16') for introducing near-infrared energy into blood present in a body part of a subject;
(b) detecting means (28) for detecting near-infrared energy emerging from the body part;
(c) positioning means (20) for positioning both the near-infrared introducing means (16,16') and the detecting means (28) closely adjacent to the body part; and
(d) processing means (34) for processing a first electrical signal produced by the detecting means (28) into a second signal indicative of the quantity of glucose present in the blood of the subject, characterised in that the processing means processes the first signal according to a formula including a temperature term $K_2 T_S$ wherein $T_S$ represents the local surface temperature of said body part and $K_2$ is a calibration constant and wherein the formula is one of

$$C = K_0 + K_1 [\log 1/I_A - \log 1/I_B] + K_2 T_S \qquad \text{(i)}$$

wherein C is concentration of glucose present in the blood, $K_0$ is an intercept constant, $K_1$ is the line slope of

$$[\log 1/I_A - \log 1/I_B],$$

and $\log 1/I_A$ and $\log 1/I_B$ each represent an optical density value at corresponding wavelengths A and B;

$$C = K_0 + K_1 [\log 1/I_A - 2*\log 1/I_B + \log 1/I_C] + K_2 T_S \qquad \text{(ii)}$$

wherein $K_1$ is the line slope of

$$[\log 1/I_A - 2*\log 1/I_B + 1/I_C],$$

$\log 1/I_C$ represents an optical density value at wavelength C, and C, $K_0$, $\log 1/I_A$ and $\log 1/I_B$ are as defined in formula (i);

$$C = K_0 + K_1 \frac{[\log 1/I_A - \log 1/I_B]}{[\log 1/I_D - \log 1/I_E]} + K_2 T_S \qquad \text{(iii)}$$

wherein $K_1$ is the line of slope of

$$\frac{[\log 1/I_A - \log 1/I_B]}{[\log 1/I_D - \log 1/I_E]},$$

$\log 1/I_D$ and $\log 1/I_E$ each represent an optical density value at corresponding wavelengths D and E, and C, $K_0$, $\log 1/I_A$ and $\log 1/I_B$ are as defined in formula (i); or

$$C = K_0 + K_1 \frac{[\log 1/I_A - 2*\log 1/I_B + \log 1/I_C]}{[\log 1/I_D - 2*\log 1/I_E + \log 1/I_F]} + K_2 T_S \qquad \text{(iv)}$$

wherein $K_1$ is the line slope of

$$\frac{[\log 1/I_A - 2*\log 1/I_B + \log 1/I_C]}{[\log 1/I_D - 2*\log 1/I_E + \log 1/I_F]}$$

$\log 1/I_C$, $\log 1/I_D$, $\log 1/I_E$ and $\log 1/I_F$ each represent an optical density value at corresponding wavelengths C, D, E and F, and C, $K_0$, $\log 1/I_A$ and $\log 1/I_B$ are as defined in formula (i).

The present invention also provides a non-invasive method for quantitatively analysing blood glucose in blood of a subject, comprising:

(a) introducing at least one pair of wavelengths of near-infrared energy from a near-infrared energy source (16,16') into blood within a body part of the subject, said pair of wavelengths being within the range of about 600 to about 1100 nanometers;
(b) detecting near-infrared energy emerging from the subject with a detector (28) which provides a first signal upon detecting energy emerging from the subject; and
(c) processing the first signal to provide a second signal indicative of the amount of glucose present in the body of the subject,

characterised in that
the first signal is processed according to a formula including a temperature term $K_2 T_S$ wherein $T_S$ represents the local surface temperature of said body part and $K_2$ is a calibration constant and wherein the formula is one of

$$C = K_0 + K_1 [\log 1/I_A - \log 1/I_B] + K_2 T_S \qquad \text{(i)}$$

wherein C is concentration of glucose present in the blood, $K_0$ is an intercept constant, $K_1$ is the line slope of

$$[\log 1/I_A - \log 1/I_B],$$

and $\log 1/I_A$ and $\log 1/I_B$ each represent an optical density value at corresponding wavelengths A and B;

$$C = K_0 + K_1 [\log 1/I_A - 2*\log 1/I_B + \log 1/I_C] + K_2 T_S \qquad \text{(ii)}$$

wherein $K_1$ is the line slope of

$$[\log 1/I_A - 2*\log 1/I_B + 1/I_C],$$

$\log 1/I_C$ represents an optical density value at wavelength C, and C, $K_0$ and $\log 1/I_A$ and $\log 1/I_B$ are as defined in formula (i);

$$C = K_0 + K_1 \frac{[\log 1/I_A - \log 1/I_B]}{[\log 1/I_D - \log 1/I_E]} + K_2 T_S \qquad \text{(iii)}$$

wherein $K_1$ is the line of slope of

$$\frac{[\log 1/I_A - \log 1/I_B]}{[\log 1/I_D - \log 1/I_E]} \; ,$$

$\log I/I_D$ and $\log 1/I_E$ each represent an optical density value at corresponding wavelengths D and E, and C, $K_0$, $\log 1/I_A$ and $\log 1/I_B$ are as defined in formula (i);

$$C = K_0 + K_1 \frac{[\log 1/I_A - 2*\log 1/I_B + \log 1/I_C]}{[\log 1/I_D - 2*\log 1/I_E + \log 1/I_F]} + K_2 T_S \qquad \text{(iv)}$$

wherein $K_1$ is the line slope of

$$\frac{[\log 1/I_A - 2*\log 1/I_B + \log 1/I_C]}{[\log 1/I_D - 2*\log 1/I_E + \log 1/I_F]} \; ,$$

$\log 1/I_C$, $\log 1/I_D$, $\log 1/I_E$ and $\log 1/I_F$ each represent an optical density value at corresponding wavelengths C, D, E and F, and C, $K_0$, $\log 1/I_A$ and $\log 1/I_B$ are as defined in formula (i); or

$$C = K_0 + K_1 \frac{[\log 1/I_A - \log 1/I_B]}{[\log 1/I_D - \log 1/I_E]} + K_2 T_S + K_3 T_A \qquad \text{(v)}$$

wherein $K_1$ is the line of slope of

$$\frac{[\log 1/I_A - \log 1/I_B]}{[\log 1/I_D - \log 1/I_E]}$$

$K_3$ is a calibration constant, $\log 1/I_D$ and $\log 1/I_E$ each represent an optical density value at corresponding wavelengths D and E, $T_A$ represents the ambient temperature of said instrument, and C, $K_0$, $\log 1/I_A$ and $\log 1/I_B$ are as defined in formula (i).

Some of these inventive methods utilize the principle of near-IR transmission while others utilize the principle of near-IR interactance.

The analysis instrument may be comprised of a lightweight, hand-held instrument including a base housing unit and a replaceable cartridge means which engages the base unit. The replaceable cartridge includes a memory device for storing data associated with a particular patient and the particular base unit, and may also include the power source for the analysis instrument. The data stored in the replaceable cartridge may include a series of glucose readings and calibration constants custom calibrated for the individual user.

Associated with the present invention, is a low-cost method and means for providing custom calibration for near-infrared instruments for measurement of blood glucose comprises obtaining a plurality of blood samples from an individual at a predetermined time interval and for a predetermined period of time. Blood glucose measurements for each blood sample are obtained and are entered into the near-infrared instrument. Non-invasive near-infrared optical absorption measurements are concomitantly taken through a body part of the individual at a second predetermined time interval and are recorded in the analysis instrument. Calibration regression analysis is then performed utilizing means for linearly interpolating the blood sample glucose measurements with the near-infrared optical measurements to custom calibrate the near-infrared instrument for the individual.

When carrying out the present invention calibration errors resulting from changes in an individual's skin temperature may be compensated. This technique involves producing a change in the individual's skin temperature while obtaining the non-invasive near-infrared blood glucose measurements. The individual's skin temperature is thereby forced to have a meaningful temperature range during the calibration process.

Embodiments of the present invention will now be described by way of example only, with reference to the accompanying drawings, in which:-

FIG. 1 is a partially schematic elevational view of a near-infrared quantitative blood analysis instrument to which the present invention pertains;

FIGS. 2A and 2B are partially schematic elevational views of alternate embodiments of near-infrared quantitative analysis instruments;

FIG. 3 is an elevational view of a location device for use with the instrument shown in FIG. 1;

FIG. 4 illustrates one embodiment for practicing the inventive method;

FIGS. 5A and 5B illustrate two known configurations for interposing filters in a light path;

FIG. 6 is a plot of log (1/l) versus wavelength;

FIG. 7 illustrates a wavelength search study via a plot of correlation coefficient versus wavelength;

FIGS. 8 and 9 illustrate plots of correlation coefficient versus wavelength for first derivative equations;

FIGS. 10 and 11 illustrate plots of correlation coefficient versus wavelength for second derivative equations;

FIG. 12 is a plot of log (1/l) versus wavelength which illustrates near-infrared energy absorption by water;

FIG. 13 is a plot of log (1/l) versus wavelength illustrating two measurement wavelengths which straddle a water band;

FIG. 14 illustrates that energy absorption by water varies with temperature;

FIG. 15 illustrates a difference spectra of glucose absorption of near-infrared energy in the body;

FIG. 16 is a plot of correlation versus wavelength illustrating the effect of a temperature term in the linear regression equation;

FIG. 17 is a plot of mid-wavelength and correlation versus gap between wavelength "A" and "B";

FIGS. 18A-D illustrate classes of peaks which correspond to the shape of a correlation versus wavelength curve;

FIG. 19 illustrates a non-invasive glucose measurement instrument according to another embodiment of the present invention;

FIG. 20 illustrates a non-invasive glucose measurement instrument according to another embodiment of the present invention;

FIG. 21 illustrates a hand-held non-invasive glucose measurement instrument according to another embodiment of the present invention;

FIG. 22 illustrates a bottom view of the non-invasive glucose measurement instrument of FIG. 21;

FIG. 23 shows a schematic representation of a replaceable cartridge according to the present invention;

FIGS. 24A-D are plots of official laboratory glucose values versus near-IR instrument glucose values illustrating potential extrapolation errors;

FIG. 25A and 25B show different sized finger inserts for the glucose measurement instrument;

FIG. 26 shows an interactance type non-invasive glucose measurement instrument according to one embodiment of the present invention;

FIG. 27 shows a side view of the interactance instrument of FIG. 26.

FIG. 28 is a flow diagram illustrating the method for custom calibrating a near-infrared analysis instrument for the measurement of blood glucose levels according to the present invention;

FIG. 29 shows a schematic representation of custom calibration instrumentation according to another aspect of the present invention;

FIG. 30 shows a schematic representation of custom calibration instrumentation according to another aspect of the present invention; and

FIG. 31 is a graph showing near-infrared energy absorption by water as a function of temperature.

In accordance with one embodiment, the invention uses the principle of light interactance to measure blood glucose level non-invasively by locating an optical transmitter and a detector on the skin surface near either an artery or a vein. Alternatively, the invention uses the principle of light transmission through a portion of the body that has relatively uniform profusion of blood in order to measure blood glucose non-invasively.

In general, the arteries and veins of the human body are buried deep in the body to protect them from possible harm. However, in certain locations of the body, these blood-carrying vessels are close to the skin surface. This is particularly true for veins. Some examples of such locations are at the crease of the elbow, the wrist, the back of the hand, and the bridge of the nose. Since the concentration of glucose is relatively constant in both the veins and arteries, valid measurements can be obtained in either. However, because veins are generally closer to the skin's surface, they usually are the better candidate for non-invasive measurements.

The finger-tip is another site particularly well suited for performing blood measurements with near-IR light. The blood supply is distributed within the finger tip and, thus, small variations in the placement of a near-IR emitter or detector will not have a profound effect on the measurement results.

According to one embodiment of the invention utilizing near-IR interactance analysis techniques, near-IR light energy at bandwidths centering on one or more wavelengths of interest is passed through the skin and connective tissues and into a blood vessel of a subject. A portion of the energy re-emerges from the blood vessel of the test subject and is detected by a detector. Following amplification of the detector-generated signal, the amplified, output is processed into an output signal indicating the amount of glucose in the subject's blood. The output signal drives a display device for providing a visual display of blood glucose content.

According to another embodiment of the invention utilizing near-IR transmission analysis techniques, near-IR light energy at bandwidths centering on one or more wavelengths of interest is transmitted through a blood-containing portion of the body of a test subject. The near-IR energy emerges from the test subject, generally opposite from the near-IR source, and is detected by a detector. Following amplification of the detector-generated signal, the amplified output is processed into an output signal indicating the amount of glucose in the subject's blood.

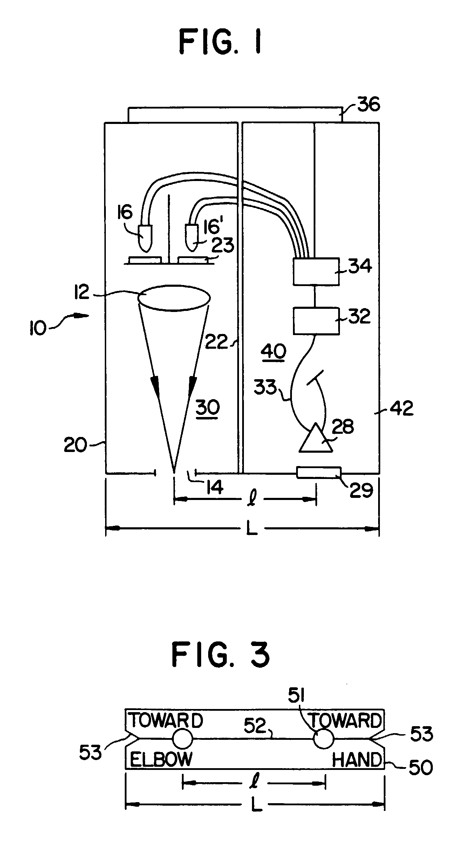

In one embodiment utilizing near-IR interactance, the entire analytical instrument, including near-infrared source, transmitter, detector, amplifier, data processing circuitry and readout is contained within a lightweight hand-held unit. See Fig. 1. Infrared emitting diodes (IREDs) disposed in one chamber of the unit are focused to transmit near-IR energy of preselected wavelength(s) to, e.g., a prominent vein of the wrist. The near-IR energy interacts with the constituents of the venous blood and is re-emitted from the vein. A detector housed within a second chamber of the unit is disposed along the vein a distance ($\ell$) from the emitter and collects this energy. The detected signal is amplified and data processed into a signal indicative of the amount of glucose in the blood. This signal is then fed to a readout device (preferably a digital readout) for recordation by a technician or direct analysis by a physician or the subject.

Other near-IR apparatus, such as the optical probe and associated instrumentation described in US-A-4,633,087 (Rosenthal), are useful in the practice of the present methods in which near-IR interactance is used to quantitatively measure blood glucose levels.

This embodiment can utilize a location device specially adapted to permit the user to locate the interactance instrument discussed above accurately along a vein. The location device permits the skin to be marked to ensure that repeated measurements are taken from the same location.

In the lightweight, hand-held interactance analysis instrument 10 illustrated in Fig. 1, included is one or more means for providing at least one point source of near-infrared energy of a predetermined bandwidth of interest which is positioned within a first chamber 30 of the instrument 10. The near-infrared point source means is positioned so that near-infrared energy being emitted from the point source means will be focused by lens 12 through window 14 and onto the skin of the test subject. The near-infrared point source means may comprise one or a plurality of infrared emitting diodes (IREDs). Two such IREDs 16, 16' are visible in the embodiment illustrated in Fig. 1. In other embodiments employing a plurality of IREDs, three, four or more IREDs may be utilized as the point source means.

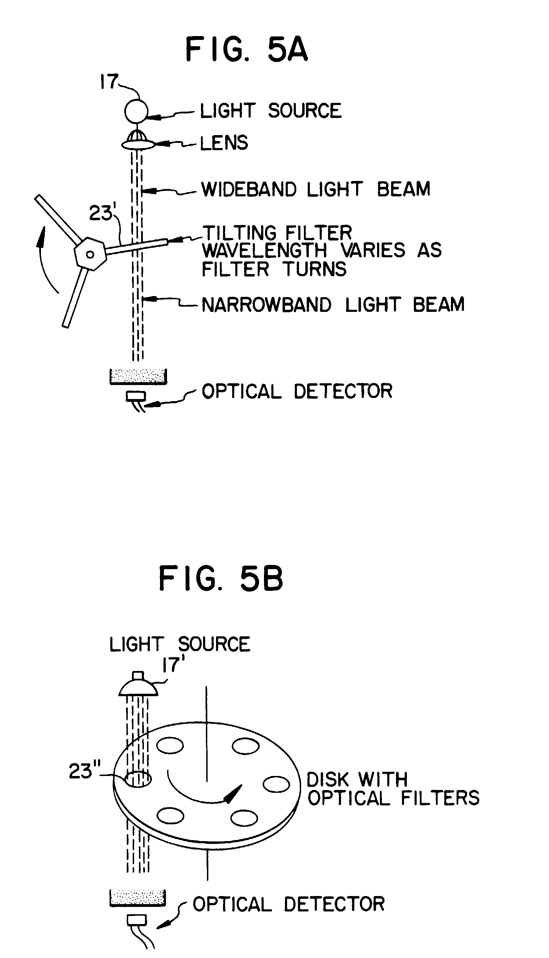

In lieu of laborious characterization and sorting of each IRED, narrow bandpass optical filters (as shown schematically in Fig. 1) can be provided between the infrared emitting diodes and the lens 12. According to this embodiment, a filter 23 is positioned between each IRED and lens 12 for filtering near infrared radiation exiting each IRED and thereby allowing a narrow band of near-infrared radiation of predetermined wavelength to pass through the filter and lens 12. Utilization of narrow bandpass optical filters provides for specific wavelength selection independent of the center wavelengths of the particular infrared emitting diodes being used. Measurements can be taken inside the half power bandwidth of the IREDs, or alternatively, outside the half power bandwidth of the IREDs as disclosed in US-A-4,286,327. Figs. 5A and 5B illustrate two other known configurations for interposing filters 23' and 23", respectively, in a light path. The light source in Figs. 5A and 5B can be either a light bulb 17 or 17', respectively, or one or more IREDs.

An optical detector, illustrated schematically in Fig. 1 and designated by reference numeral 28, is disposed within a lower end portion 42 of a second chamber 40 in case 20. Inner wall 22 is positioned between detector 28 and illumination section 30, thereby providing an optically-isolating mask which prevents near infrared radiation from the point source means and/or lens 12 from impinging directly on detector 28. The near-infrared optical detector 28 generates an electrical signal when near-infrared radiation is detected thereby.

The optical detector 28 is connected to the input of an electrical signal amplifier 32 by suitable electrical conducting means 33. Amplifier 32 may be an inexpensive integrated circuit (IC) signal amplifier, and amplifies the signals generated when near-IR energy strikes detector 28. The output of amplifier 32 is fed to a controller/data processor and display driver 34 which provides a signal to readout device 36. The readout device 36 may have a digital display for directly displaying the amount of glucose present in the subject's blood.

The embodiment of Fig. 1 includes an optical filter 29 for shielding all but the desired near-IR energy from detector 28. Filter 29 and window 14 are positioned for direct contact with the skin of the test subject. An optically clear window can be employed in lieu of filter 29, if desired, and in lieu of the window opening 14.

As noted earlier, the embodiment illustrated in Fig. 1 utilizes the principle of near-IR interactance for quantitative analysis. In interactance, light from a source is shielded by an opaque member from a detector so that only light that has interacted with the subject is detected.

In use, the analysis instrument 10 is positioned so that its flat bottom surface rests on the skin directly above the prominent vein of the wrist of a test subject. Light at the selected wavelengths emerging from the instrument interacts with venous blood of the subject and is detected by detector 28. Detector 28 generates an electrical signal which is processed as described above.

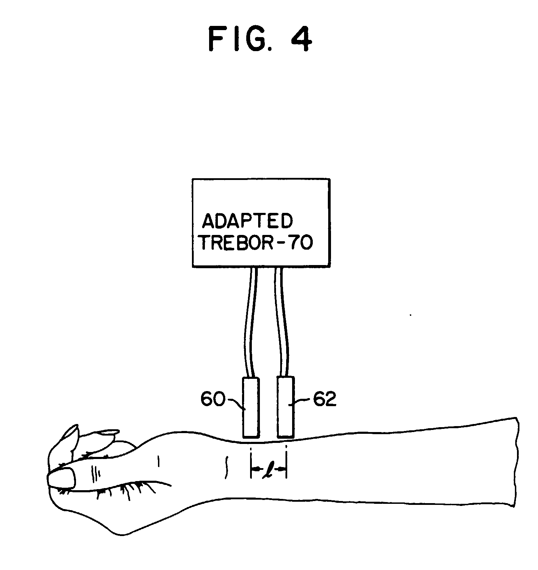

Accurate analysis is facilitated when the user locates the transmitter and detector filter (or window) directly over the prominent vein of the wrist. The location device illustrated in Figure 3 simplifies this procedure. The device 50 is constructed of, e.g., a plastic material and has an overall length $L$ equal to the length $L$ of the analysis instrument 10 of Figure 1. Two holes 51 are present in the device and are located in the same relation as 14 and 29 in Figure 1, on midline 52, a distance $\ell$ apart corresponding to the distance $\ell$ of Figure 1. The holes 51 permit observation of the prominent vein. When the device is placed on the wrist and the vein is centered in each hole 51, the wrist is marked (e.g. with a felt-tipped pen) at notches 53. The location device is then removed and replaced by the analysis instrument 10 with assurance that the instrument is properly located over the vein.

An alternate procedure for practicing the inventive method is accomplished by the use of fiber optic light probes as seen in Figure 4. These probes are connected with a near-IR analysis instrument such as the commercially available TREBOR-70 scanning spectrophotometer which has been adapted to process a signal for glucose analysis. A probe 60 is placed over the prominent vein and transmits near-IR energy of the desired wavelength(s). The near-IR energy interacts with the blood constituents and is collected by a second probe 62 placed over the vein a short distance $\ell$ from first probe 60. A detector associated with the analytical instrument provides an electrical signal which is processed, as described above, to reveal quantitative information concerning blood glucose.

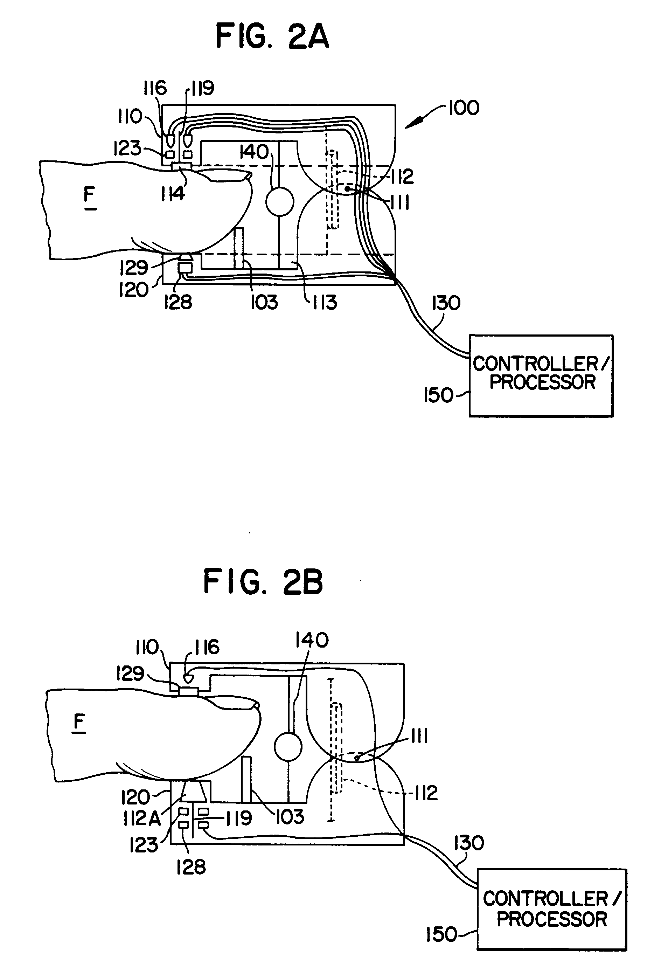

It has been found that accurate quantitative analysis of blood glucose levels can be made at a variety of wavelengths with both interactance and transmittance technologies. In presently preferred embodiments illustrated in Figures 2A and 2B, near-IR light energy is transmitted through the finger of the test subject and then detected by an optical detector. As in the above described embodiments, a combination of measurement wavelengths is selected which emphasizes the glucose absorption and removes the effect of interfering absorption, for example, due to water, fat and protein.

In the embodiment shown in Figure 2A, a near-IR probe 100 is adapted to be placed over the finger F of a test subject and in this particular embodiment includes a point source means of near-IR light energy comprised of at least two IREDs 116 disposed within an upper flange 110. Each IRED is paired with a narrow bandpass optical filter 123 and is optically isolated via opaque light baffle 119. The inwardly-facing surface of flange 110 is provided with an optional optically clear window 114 for placement against the subject's finger.

Upper flange 110 is hinged about shaft 111 to lower flange 120, and a spring 112 serves to maintain the flanges in a closed position. An optical detector 128 is disposed in lower flange 120 opposite the near-IR source 116. The detector is disposed behind an optional window 129 which can be constructed of a material which is either optically clear or which excludes interfering light yet permits the desired near-IR light to pass. A finger stop 103 helps place and maintain the subject's finger in its proper position within the probe 100. Each of the flanges is provided with light-shielding barriers 113 (shown in phantom in Figure 2A) to block ambient light from entering the probe.

In this embodiment the IREDs are pulsed, i.e., energized in sequence, so that the detector 128 receives light transmitted from only one of the IREDs at any one time. This pulsed IRED technology is described in U.S. US-A-4,286,327 which is incorporated herein by rererence. In other similar embodiments a group of IREDs (and optional narrow bandpass filters) with substantially identical center wavelength output can be pulsed.

Probe 100 is an electrical connection with a processor unit which is schematically illustrated in Figure 2A. The processor unit houses a power source, signal amplifying, data processing and display circuitry as described in connection with the embodiment of Figure 1 and standard in near-IR analysis instrumentation.

An alternate embodiment is seen in Figure 2B. Here, probe 110 includes a single constant output IRED 116 installed behind an optional window 129. Light transmitted through the finger is gathered by optical funnel 112A, which is constructed of a transparent or translucent material, and detected by multiple detectors 128. The detectors are optically isolated from one another by opaque light baffle 119. Each detector is paired with a narrow bandpass optical filter 123 and thus is set up to detect only light within the narrow wavelength range of its filter.

Near-IR point source means 116 can consist of one or more IREDs of known bandwidth and center frequency output or, as described above, can include a narrow bandpass optical filter within the light path to provide for the detection of only those wavelengths which are of interest. Multiple wavelengths can be utilized in transmission analysis and can be generated via multiple IREDs provided they are consecutively illuminated. Another approach is to use a single IRED with multiple bandpass filters which are mechanically moved through the light path as seen in Figures 5A and 5B. A third approach uses a single or group of IREDs capable of emitting a plurality of desired wavelengths with the use of multiple optical filters, each filter being associated with a respective detector. Single IREDs which emit two, three or four narrow bandwidths are commercially available.

In use, the finger of the test subject is inserted between the flanges 110 of the probe 100. Near-IR light energy is emitted by the point source means, is transmitted through the finger and is detected by optical detector 128. The electrical signals produced by the detectors are transmitted via line 130 to a controller/processor unit 150 where the signal is amplified and data processed using a suitable algorithm as described below. Blood glucose level is displayed on a readout device which preferably includes a digital display.

Accurate measurements of the concentration of blood glucose can be made using near-IR quantitative analysis algorithms which have only a single variable term, such as the following:

Approximated First Derivative Algorithm

$$C = K_o + K_1 [\log 1/I_G - \log 1/I_H]$$

Approximated Second Derivative Algorithm

$$C = K_o + K_1 [\log 1/I_A - 2*\log 1/I_B + \log 1/I_C]$$

Normalized First Derivative Algorithm

$$C = K_o + K_1 \frac{[\log 1/I_G - \log 1/I_H]}{[\log 1/I_I - \log 1/I_J]}$$

Normalized Second Derivative Algorithm

$$C = K_o + K_1 \frac{[\log 1/I_A - 2*\log 1/I_B + \log 1/I_C]}{[\log 1/I_D - 2*\log 1/I_E + \log 1/I_F]}$$

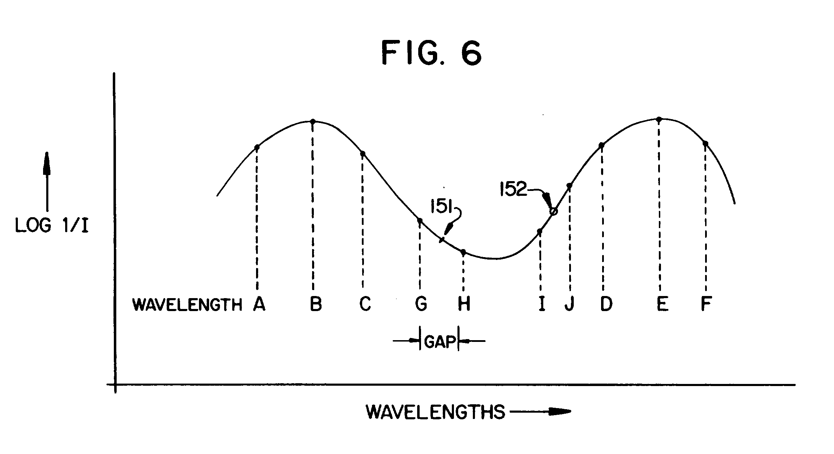

where C denotes concentration of glucose present in the blood, $K_0$ is the intercept constant, $K_1$ is the line slope of the variable term, and the log 1/I terms each represent an Optical Density (O.D.) value at a particular wavelength. In Fig. 6, an example of an overall absorbance curve for a test subject is shown, wherein log 1/I (O.D.) values for the above algorithms are plotted. In Fig. 6, optical energy is absorbed at wavelength B proportional to the constituent being measured, and optical energy is absorbed at wavelength E proportional to the total substance being measured. Points 151 and 152 are first derivative midpoints. The distance between, for example, wavelength G and wavelength H is referred to herein as the "gap" between two wavelengths. It has been found that a plurality of wavelength pairs, all centered on the same wavelength, can be used in the above algorithms. These algorithms are easily programmed into suitable microprocessor circuitry by those skilled in the art. The use of these single variable term equations is highly desirable because it allows simplified instrument calibration, thereby allowing the production of low-cost instruments.

The intercept constant $K_0$ and the slope constant $K_1$ are determined by individually calibrating each unit.

Another class of usable near-IR standard algorithms involves the use of multiple regression terms. Such terms can be individual log 1/I terms or can be a multiple number of first or second derivative terms with or without a normalizing denominator. Such multiple terms may provide additional accuracy, but introduce higher calibration expense which results in a more expensive instrument.

Data on a plurality of physical parameters of the test subject can also be utilized in conjunction with multiple wavelength measurement of near-infrared interactance, as in prior US-A- 4,633,087, to improve the accuracy of the present blood glucose measurements.

Selection of combinations of wavelengths which emphasize glucose absorption and remove possible interfering absorptions can be performed by computer search studies. In general, a suitable combination of wavelengths will include at least one wavelength which is sensitive to blood glucose, and at least one wavelength which is insensitive to blood glucose (reference wavelength). The following examples show results of wavelength search studies, which are provided herein for illustrative purposes only, and are not to be construed in a limiting sense.

Example I

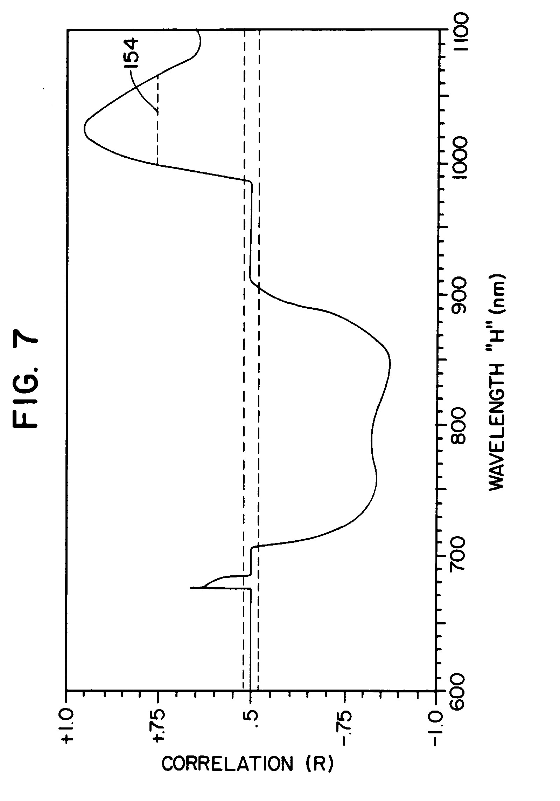

Figure 7 presents correlation coefficient versus wavelength data from a search study utilizing an approximated first derivative algorithm as defined above, and illustrates that the use of the wavelength pair of 980 ± (plus and minus) 35 nm provides a high correlation between blood glucose and absorption of near-IR energy at those two wavelengths. Figure 7 utilizes the above approximated first derivative algorithm, wherein G and H are as shown in Figure 6, and equal to 945 nm and 1015 nm, respectively. Thus, in this example, the "gap" is 70 nm (1015 nm - 945 nm). The number of samples tested was 30 in this case. The value of $K_0$ in the approximated first derivative algorithm is 196.9 and for

$K_1$ is 4,802.6. In this case, the standard deviation was 13.54, with a correlation of +0.948. Reference numeral 154 of Figure 7 indicates a range of candidates for wavelength H with a "gap" equal to 70 nm and a "smoothing" factor of 41. "Smoothing" is the modification of data derived from a scanning spectrophotometer in order to simulate the results which would be obtained at the half power bandwidth of optical filters. "Smoothing" involves taking log 1/I data at an equal number of wavelengths above and below each wavelength of interest and averaging the results. Thus, with a "smoothing" value of 41, data is taken at 20 wavelengths above and 20 wavelengths below the bandwidth of interest, in addition to each wavelength of interest. An example of one embodiment of the invention uses IREDs which provide near-IR energy at two frequencies which are, respectively, equidistant above and below approximately 980 nm, i.e., they can be represented by the formula $980 \pm x$ nm. The value of $x$ is not unduly critical so long as the two frequencies are centered on approximately 980 nm. A suitable value for $x$ can be, for example, a number from 10 to 40.

## Example II

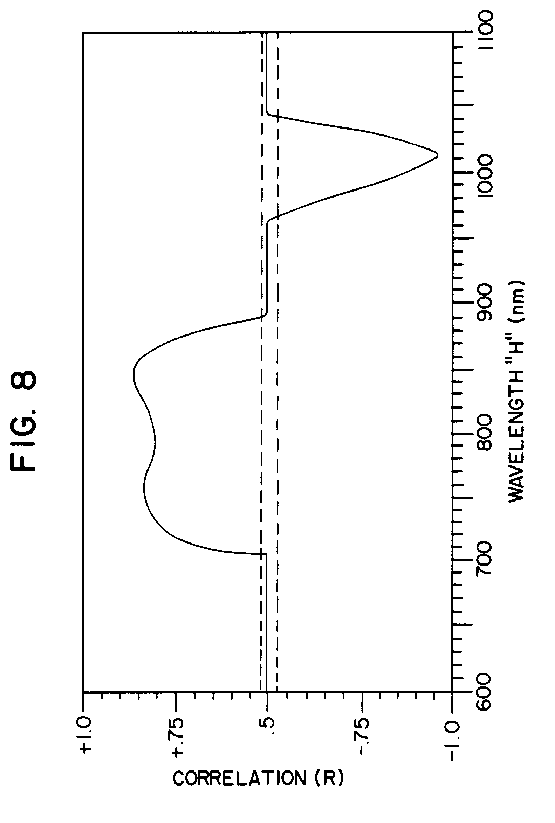

Figure 8 shows that a suitable wavelength for a numerator in the above normalized first derivative algorithm is approximately 1013 nm (i.e., 980 nm + 35 nm) wherein $K_0 = 296.8$, $K_1 = -175.6$, "gap" G-H: 70 nm, wavelength J: 915 nm, "gap" I-J:20 nm, standard deviation = 12.21 and correlation = - 0.958 (30 samples).

## Example III

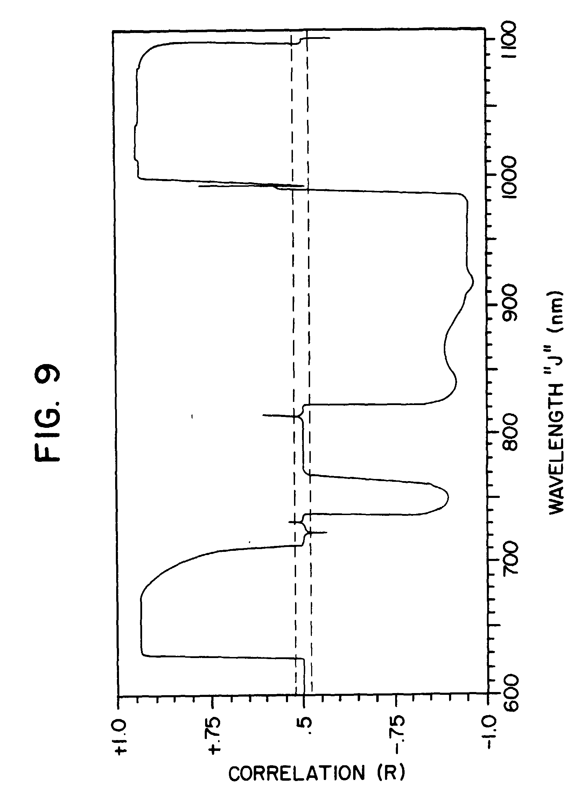

Figure 9 shows that there are many wavelength regions that can provide midpoint wavelengths for use in the denominator of the above normalized first derivative algorithm when the numerator utilizes $980 \pm 35$ nm wavelengths, wherein $K_o$, $K_1$, "gap" G-H, gap I-J, standard deviation, correlation and sample size are the same as in Example II and Figure 8, and wherein wavelength H is 1013 nm. Examples of such wavelength regions are seen to be from 610 to 660 nm, from 910 to 980 nm and from 990 to 1080 nm.

## Example IV and V

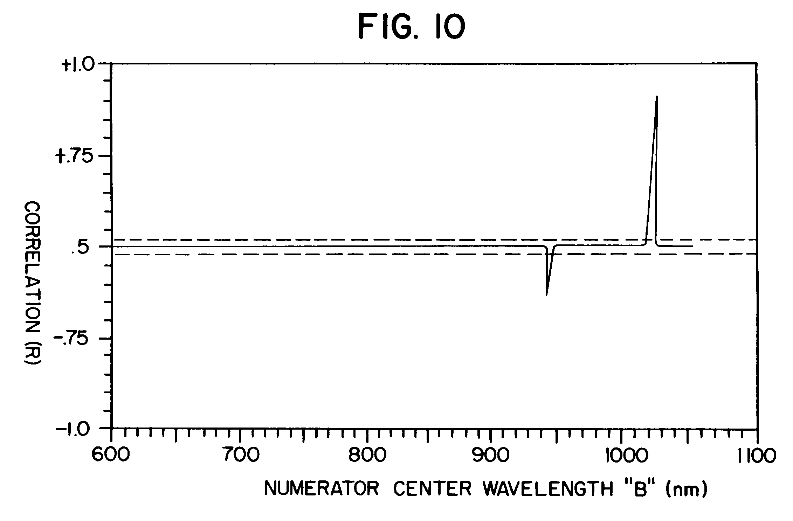

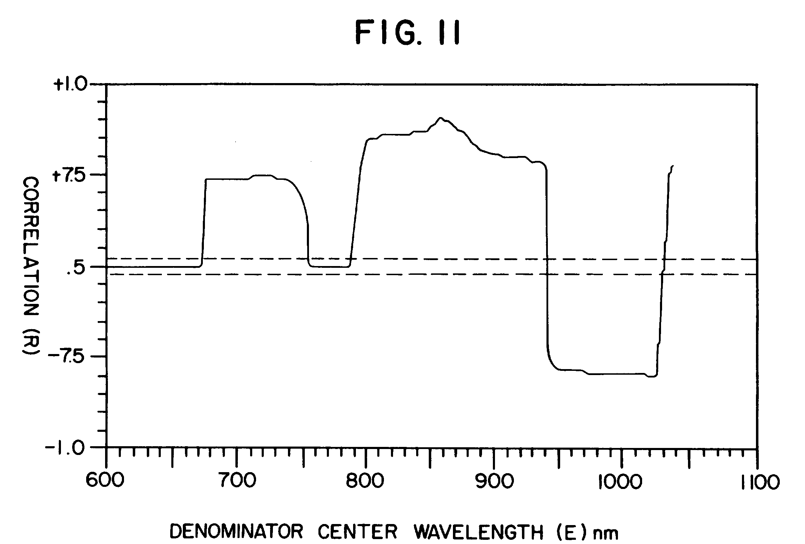

Figures 10 and 11 illustrate optimum center wavelengths for use in the normalized second derivative algorithm described above. Figure 10 is a plot of correlation coefficient versus wavelength which shows that a suitable numerator center frequency is approximately 1020 nm, wherein in the above normalized second derivative algorithm, $K_o = 205,856$, $K_1 = 356.457$, "gap" A-B and B-C = to 53 nm, wavelength E: 850 nm, "gap" D-E and E-F = to 68 nm and standard deviation = 20.44 (47 samples). Figure 11 shows that a denominator center frequency of about 850 nm is suitable, wherein $K_o$, $K_1$, "gap" A-B and B-C, "gap" D-E and E-F, standard deviation, and sample size are as in Figure 10, and wherein wavelength B is 1020 nm.

The accuracy of the preferred near-IR transmission embodiments shown in Figs. 2A and 2B can be further improved by altering the algorithm to include finger thickness as a parameter. According to Lambert's law, energy absorption is approximately proportional to the square of the thickness of the object. The thickness of the test subject's finger can be quantified by installing a potentiometer 140 between the flanges of the probe 100 as seen in Figures 2A and 2B. The output of the potentiometer, which is in electrical connection with the data processing circuitry, is indicative of finger thickness. A non-linear potentiometer can approximate the $T^2$ value via its output alone so that a separate squaring calculation step is not necessarily required.

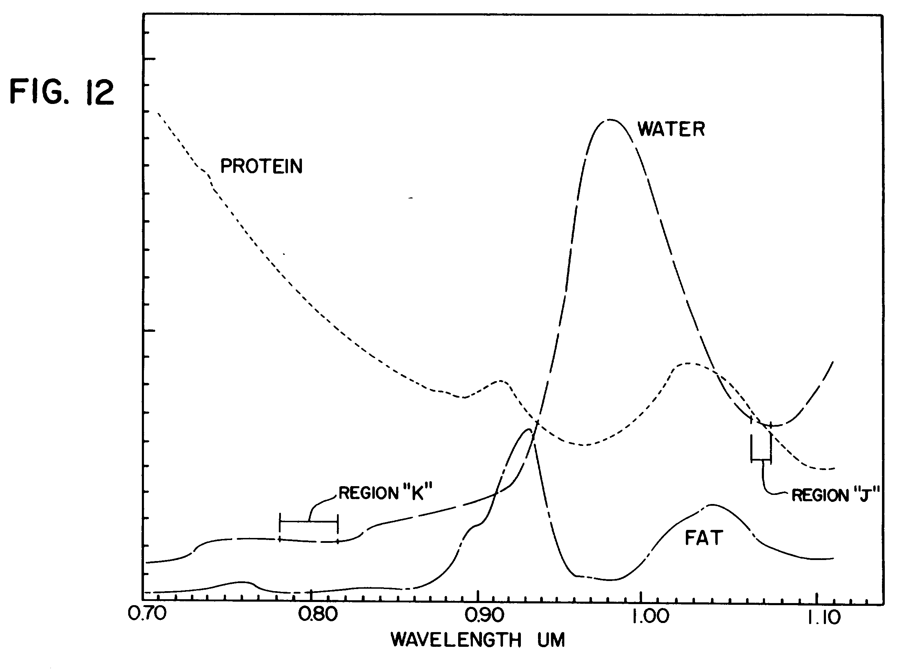

Selection of suitable combinations of measurement wavelengths can take into account the identification and removal of possible interfering absorptions, such as due to water. Figure 12 illustrates that water is one of the strongest absorbers of near-IR energy. The effect of this inherent strong absorption is magnified by the very high water content in the human body (i.e., approximately 60% for the average person). Thus, measurements in the near-infrared must be performed with care to avoid being distorted due to slight changes in body water caused by either dehydration (e. g., perspiration) or due to excessive intake of fluids.

This care is particularly critical when trying to measure blood glucose. This is because of the extremely low concentration of glucose level in the blood (average of approximately 100 milligrams per deciliter).

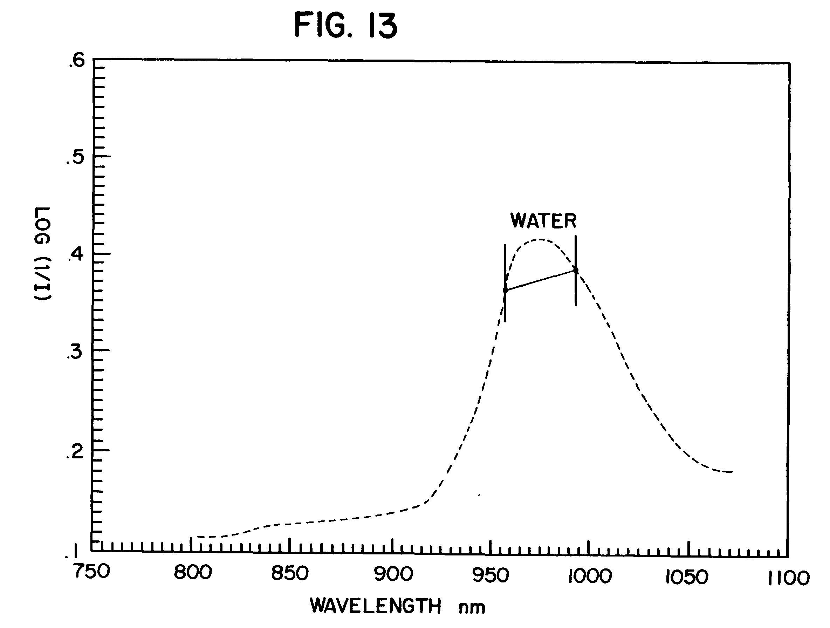

The wavelength search studies discussed above illustrate that where the mid-wavelength between the two measurement wavelengths would be approximately at the center of a water "dead band" (i.e., minimal interference due to water), good measurement has been provided (see Figure 7). The reason for this is that these two measurement wavelengths straddle the water "dead band," and thereby minimize errors due to the change in water content. Figure 13 illustrates that the two measurement wavelengths which straddle the water band correspond to the wavelength pair in Figure 7 which provides a high correlation between blood glucose and absorption of near-IR energy.

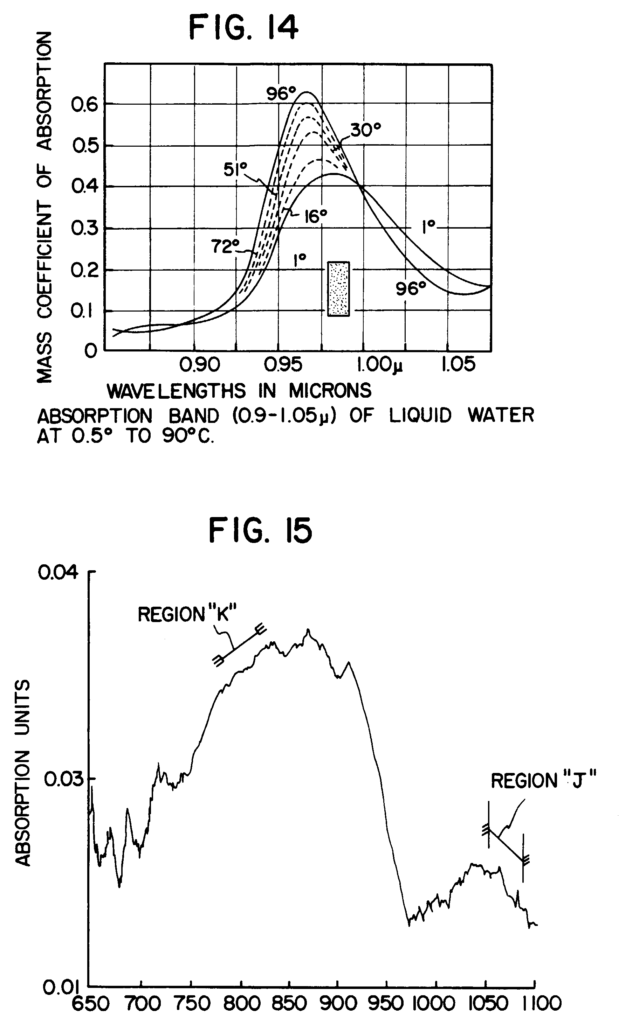

However, Figure 14 illustrates the well-known phenomenon that the water absorption curve varies with change in temperature. Since temperature at different extremities on the body can vary for a variety of reasons, illness or ingestion of cold or hot fluids/foods, the potential for error in glucose measurement made where the wavelength pair straddles the water band may become excessive.

To overcome this potential temperature limitation, two regions of the spectrum have been discovered that appear to be well suited for measurement of blood glucose. These are indicated as Region "J" and Region "K" in Figure 12 for the standard near-IR absorption curve for water. In Region "J", the water absorption curve reaches a minimum near 1070 nm. In this region, changes in water concentration would have the least effect of any place in the longer wavelength spectrum.

Figure 15 illustrates the effective spectra of glucose in the human body. This spectra was developed by subtracting two spectra obtained by transmitting optical energy at 1 nm intervals between 600 to 1100 nanometers through the distal portion of the index finger. The two scans were made approximately one hour apart during which time the subject drank a concentrated solution of dextrose, thereby raising the blood glucose level. Thus, this difference in spectra in Figure 15 is primarily due to glucose.

As shown in this figure, there is a broad absorption due to glucose that occurs between 850 and 900 nm as well as another one that occurs at approximately 1050 nm. Although other absorptions are occurring, they are not as well defined.

The glucose absorption that occurs at approximately 1050 nm causes a negative slope between approximately 1070 to 1100 nm (see Figure 12, Region "J"). This negative slope is measured by using two log 1/I measurements, for example, one at approximately 1070 nm and the other one at approximately 1090, and coincides favorably with the nadir point on water absorption curve (i.e., Region "J", Figure 12). Thus, a change in body water level would have minimal effect on the glucose reading in this area.

As stated above, one of the parameters affecting near-infrared accuracy is change in temperature of the subject being measured. Although there appears to be little actual relationship between finger temperature and glucose level, it has been discovered that by adding a temperature term to the regression equation, a significant improvement in correlation coefficient occurs. The temperature term can be added to any of the near-IR quantitative analysis algorithms identified as providing accurate measurements of concentration of blood glucose, such as the following:

$$C = K_o + K_1 [\log 1/I_A - \log 1/I_B] + K_2 T_S$$

where C denotes concentration of glucose present in the blood, $K_o$ is the intercept constant, $K_1$ is the line slope of the variable term, $K_2$ is a calibration constant, the log 1/I terms each represent an Optical Density (O.D.) value at a particular wavelength and $T_s$ represents the local surface temperature of the body where the optical reading is being made. The temperature term $T_s$ can also be added to the algorithms for three, four or six wavelengths described above.

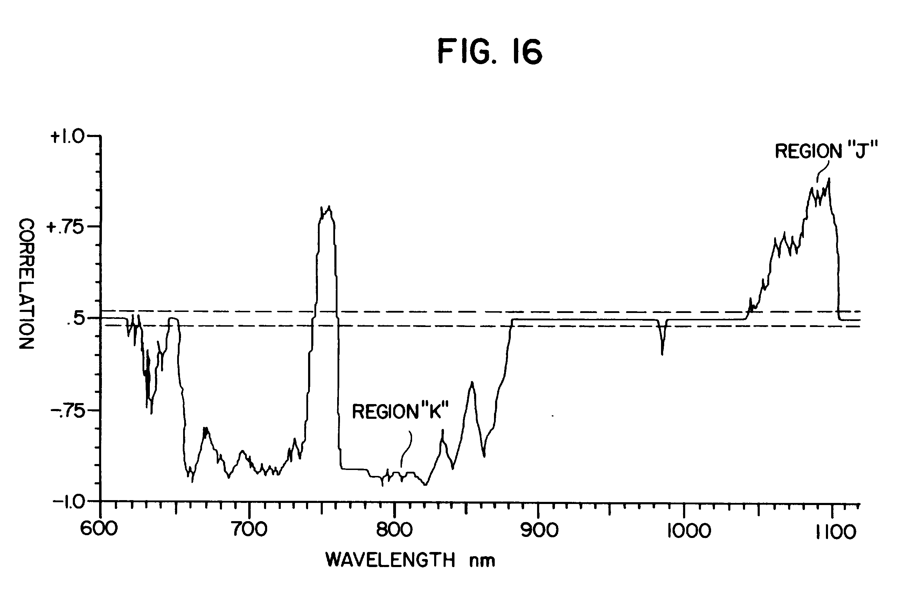

Figure 16 illustrates the improved correlation coefficients obtained in the Region "J" band using the temperature term $T_s$ in the linear regression equation. Although the correlation in Region "J", approximately 1080 nm, is not the highest of any wavelength, it is of a level usable for a commercial instrument, particularly if consideration is given to potential errors introduced due to change in body water level.

In addition to the Region "J" band, the Region "K" band provides a unique measurement candidate for glucose. Figure 12 shows that the water curve has no significant slope from 780 nm to 810 nm (Region "K"). This water "dead band" provides a good wavelength region to measure glucose, as shown by the relatively steep slope of glucose in this region (see Figure 15).

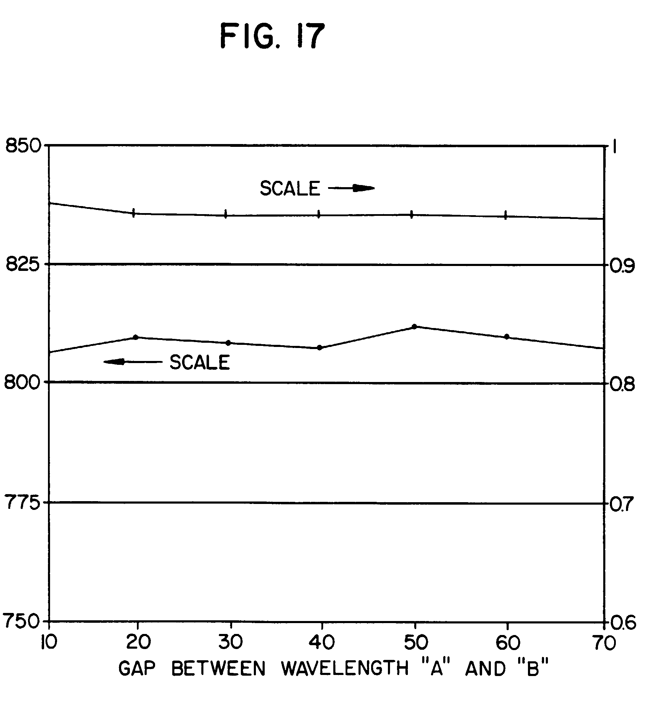

Figure 17 further demonstrates the uniqueness of this Region "K". It shows that the "mid-point" wavelength (i.e., the wavelength halfway between wavelength "A" and wavelength "B") remains essentially constant independent of gap size. This is due to the "dead band" (i.e., no interference due to water). The importance of this Region "K" is further demonstrated by the fact that the correlation coefficient remains high (between 0.94 and 0.95) independent of gap size.

Another parameter affecting near-infrared analysis accuracy is ambient temperature change within the measurement instrument. It is known that the output of silicon optical detectors (the detector of choice in the spectrum regions of 600 to 1100 nm) is extremely sensitive to temperature changes. Thus, varying the "room temperature" could have negative effect on measurement reliability. By adding a third term to the regression equation, i.e., an ambient temperature term, a significant improvement in the correlation coefficient occurs. The ambient temperature term can be added to any of the near-IR quantitative analysis algorithms identified above, such as the following:

$$C = K_o + K_1 [\log 1/I_A - 2*\log 1/I_B] + K_2 T_S + K_3 T_A$$

wherein each term described above is the same, $K_3$ is a calibration constant and $T_A$ represents the ambient temperature within the measurement instrument. The ambient temperature term can also be added to the algorithms for three, four or six wavelengths as described above.

In a preferred embodiment, a thermistor is used to measure the instrument's ambient temperature. Although alternative means could be used, a thermistor is the most practical solution for a battery powered instrument.

Accurate measurements of the concentration of blood glucose can be made using any suitable algorithm described above utilizing either two, four or six wavelengths. The two-wavelength model provides the lowest-cost instrument since only two optical measurements need be made. The applicability of the two-wavelength model has been verified by repeatedly testing a group of diverse people, such as those of different racial origins, sex, body fat and those without a history of diabetes. These tests demonstrated high correlation coefficients obtained (e.g., typical correlation coefficients exceed 0.93). Further, tests indicated that wavelengths "A" and "B" which provided the highest correlation were substantially different for each person.

This "wavelength uniqueness" for each person directly results from the individual's organic body composition. The human body is primarily composed of water (55% to 70%), fat (5% to 40%), and protein (5% to 15%). These three constituents add up to more than 95% of the total body composition. Since water, fat and protein all have strong near-IR absorption bands, the measurement of glucose (concentration of less than 0.1% of the body) must be made at wavelengths that have minimum interference by these major near-IR absorptions.

Since each individual has a different relative body composition, the minimum interference points where glucose can be measured differs greatly between individuals. In some instances, this change in wavelength can be as much as 50 nm. Tables I and II reproduced below illustrate this difference. The tables show the correlation coefficient versus measurement wavelength for three different individuals. A constant difference between A and B of 10 nm was retained for the measurements. As shown in Table I, Subject A's optimum "B value" was at 820 nm, where Subject B's optimum "B value" was at 790 nm, and Subject C's optimum "B value" was at 840 nm.

| | Table I FIGURE 18A Two Wavelength Model For Halfpower Bandwidth = 15nm | | | Table II FIGURE 18B Two Wavelength Model For Halfpower Bandwidth = 60nm | | |
|---|---|---|---|---|---|---|
| Person | R Correlation | "B" nm | GAP (A-B) | R Correlation | "B" nm | GAP (A-B) |
| A | .92 | 790 | 10 | .93 | 790 | 10 |
| A | .95 | 820 | 10 | .93 | 820 | 10 |
| A | .90 | 840 | 10 | .91 | 840 | 10 |
| B | .93 | 790 | 10 | .92 | 790 | 10 |
| B | .88 | 820 | 10 | .89 | 820 | 10 |
| B | Low | 840 | 10 | .84 | 840 | 10 |
| C | Low | 720 | 10 | Low | 790 | 10 |
| C | .88 | 820 | 10 | .84 | 820 | 10 |
| C | .97 | 840 | 10 | .86 | 840 | 10 |

The Table I measurements were made using a near-IR source having an optical half-power band width of approximately 15 nm. This half-power band width is commonly used for near-infrared measurements and it can be provided by placing a narrow bandpass optical filter in front of an infrared emitting diode.

Table II illustrates the same data as Table I except for the use of a very wide half-power band width. The half-power band width shown in Table II is equivalent to that provided by an infrared emitting diode without any optical filter (approximately 60 nm). As shown in Table II, there is an accuracy penalty incurred by using these wide band widths. However, with this approach the production of a lower-cost instrument becomes possible because the optical filters can be omitted. Further, it is recognized that, in some cases, this penalty in accuracy would be acceptable.

In the algorithms provided above, the two temperature terms $T_S$ and $T_A$ are not optical variables in that they can be measured by independent thermistors. For this reason, the constants $K_2$ and $K_3$ may not vary between instruments.

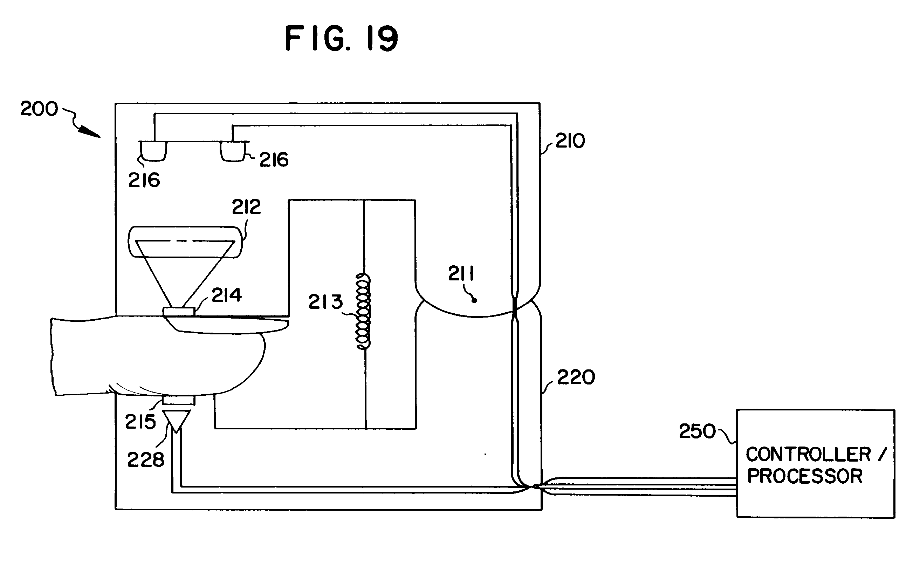

According to one embodiment of the present invention, a non-invasive blood glucose instrument is constructed which provides an accurate blood glucose level measurement correcting for inaccuracies resulting from each person's "wavelength uniqueness." In the lightweight, hand-held analysis instrument 200 illustrated in Figure 19, included is a means for providing at least one point source of near infrared energy. The near-infrared point source means is positioned so that near-infrared energy being emitted from the point source means will be focused by a lens 212 through window 214 and onto the skin of the test subject. The near-infrared energy emerging from the test subject will travel through window 215 and be detected by detector 228. Detector 228 is electrically connected to controller/processor 250 which calculates the test subject's blood glucose level. In one embodiment of the present invention, also illustrated in Figure 19, the blood glucose measurement instrument 200 is constructed having a first section 210 and a second section 220 pivotally connected together by hinge 211. Spring 212 is connected to first section 210 and second section 220 and facilitates securing the instrument against the test subject's skin, thereby substantially shielding interfering ambient light from detector 228.

In a preferred embodiment, the near infrared point source means may comprise six IREDs 216 (Figure 19 shows only two). The center wavelength of each IRED can be separated by a constant wavelength, for example, an IRED with a center wavelength at 790 nm, the next at 800 nm, etc. (i.e., center wavelengths from 790 nm to 850 nm). The use of IREDs as a light source allows a simple, low-cost instrument to be developed. Also, this instrument could contain just one IRED and utilize the configurations in Figures 5A and 5B for interposing optical filters 23' and 23", respectively, in the light path.

In the embodiment of the present invention utilizing a plurality of IREDs, each IRED is sequentially pulsed. As illustrated in Figure 19, microprocessor 250 is used to sequentially pulse each IRED. Microprocessor 250 can also be used to custom calibrate the instrument to a specific individual. This is accomplished by measuring the person at a time of low glucose level such as after an overnight fast. The person then increases his glucose concentration by, for example, drinking a dextrose solution, and is again measured. The microprocessor can be used to determine which two IREDs provide the maximum difference, and then use those IREDs in subsequent glucose measurements for that individual.

However, if better accuracy is required, optical filters could be used with the IREDs. In such a case, a "filter tray" containing a plurality of interchangeable optical filters could be made available to, for example, a medical professional. The medical professional would have a master instrument that can be used in conjunction with glucose tolerance test. This master instrument would be used to define the optimum wavelength for a specific individual. Moreover, it would define the instrument's calibration constants for that individual as well.

The medical professional could then place the correct optical filters that correspond to what was found on the master instrument into a small portable home instrument. These optical filters would be installed using the "filter tray" discussed above, thereby customizing a home instrument for that specific individual. Likewise, the calibration constants will also be entered into the instrument by the medical professional.

In another embodiment of the present invention, the four-wavelength model enables accurate glucose measurement testing and has as a major advantage that the wavelengths would not have to be varied for each individual tested. One set of wavelengths is applicable for all people.

Table III reproduced below presents a comprehensive listing of candidate wavelength combinations for this model. This data (as well as the six-wavelength model candidates) was derived from actual tests on three separate individuals, two of whom were diabetics. These tests included a minimum of 200 separate measurements on each person while their blood glucose varied considerably by undergoing glucose tolerance tests. Because of the diversity of these individuals, it is believed that the data shown is reasonably representative of the general population.

## Table III

### DELTA OD/DELTA OD; ALL REASONABLE CANDIDATES
### (R > .8)

Criteria:  Must be OK on 3 People; "$R_{min}$" is lowest
correlation coefficient of the lowest person.

| NUM "B" | GAP (A-B) | Class | Region | DEN "E" | GAP (D-E) | $R_{min}$ |
|---------|-----------|-------|--------|---------|-----------|-----------|
| 705     | 10        | 4     | D      | 650     | 30        | .91       |
| 711     | 20        | 4     | D      | 650     | 10        | .87       |
| 1091    | 20        | 3     | A      | 650     | 20        | .85       |
| 1087    | 20        | 4     | A      | 660     | 10        | .84       |
| 1090    | 20        | 3     | A      | 670     | 10        | .84       |
| 1088    | 20        | 4     | A      | 680     | 10        | .84       |
| 1088    | 20        | 3     | A      | 690     | 10        | .85       |
| 1092    | 30        | 3     | A      | 700     | 10        | .90       |
| 1089    | 20        | 4     | A      | 710     | 10        | .82       |
| 1092    | 20        | 4     | A      | 730     | 10        | .86       |
| 1089    | 20        | 4     | A      | 740     | 10        | .85       |
| 1090    | 20        | 3     | A      | 750     | 10        | .85       |
| 627     | 10        | 3     | D      | 820     | 10        | .90       |
| 676     | 10        | 4     | D      | 820     | 10        | .85       |
| 940     | 10        | 1     | Other  | 820     | 10        | .89       |
| 1093    | 20        | 3     | A      | 820     | 10        | .86       |
| 637     | 20        | 4     | D      | 830     | 10        | .82       |
| 828     | 20        | 3     | B      | 830     | 10        | .85       |
| 942     | 20        | 1     | Other  | 840     | 20        | .89       |
| 1091    | 20        | 3     | A      | 830     | 10        | .86       |
| 630     | 20        | 4     | D      | 840     | 10        | .83       |
| 960     | 20        | 1     | Other  | 840     | 10        | .85       |
| 996     | 20        | 2     | Other  | 840     | 10        | .90       |
| 1045    | 10        | 1     | Other  | 840     | 10        | .82       |
| 1091    | 20        | 2     | A      | 840     | 10        | .88       |
| 819     | 20        | 3     | B      | 850     | 10        | .85       |
| 1088    | 20        | 4     | A      | 850     | 10        | .84       |
| 627     | 20        | 3     | D      | 860     | 10        | .85       |
| 1088    | 20        | 3     | A      | 860     | 10        | .99       |
| 1089    | 20        | 3     | A      | 870     | 10        | .85       |

14

| NUM "B" | GAP (A-B) | Class | Region | DEN "E" | GAP (D-E) | $R_{min}$ |
|---|---|---|---|---|---|---|
| 1089 | 20 | 3 | A | 880 | 10 | .87 |
| 825 | 20 | 3 | B | 890 | 10 | .84 |
| 1089 | 20 | 3 | A | 890 | 10 | .85 |
| 825 | 20 | 3 | B | 900 | 10 | .85 |
| 1089 | 20 | 3 | A | 900 | 10 | .85 |
| 828 | 30 | 3 | B | 910 | 10 | .83 |
| 1088 | 20 | 2 | A | 910 | 10 | .86 |
| 837 | 30 | 2 | B | 920 | 10 | .84 |
| 1088 | 20 | 2 | A | 920 | 10 | .86 |
| 838 | 30 | 2 | B | 930 | 10 | .87 |
| 1088 | 20 | 3 | A | 930 | 10 | .85 |
| 820 | 10 | 2 | B | 940 | 10 | .87 |
| 832 | 20 | 1 | B | 940 | 10 | .87 |
| 837 | 30 | 2 | B | 950 | 10 | .87 |
| 838 | 30 | 2 | B | 960 | 10 | .84 |
| 838 | 30 | 2 | B | 970 | 10 | .87 |
| 1091 | 20 | 3 | A | 980 | 10 | .86 |
| 1091 | 20 | 2 | A | 990 | 10 | .86 |
| 1091 | 20 | 3 | B | 1000 | 10 | .86 |
| 819 | 10 | 3 | B | 1010 | 10 | .81 |
| 1088 | 20 | 2 | A | 1010 | 10 | .86 |
| 1088 | 20 | 2 | A | 1020 | 10 | .86 |
| 1088 | 20 | 2 | A | 1030 | 10 | .86 |
| 1088 | 20 | 2 | A | 1040 | 10 | .86 |
| 1088 | 20 | 2 | A | 1050 | 10 | .86 |
| 1088 | 20 | 2 | A | 1060 | 10 | .86 |
| 705 | 20 | 4 | C | 1070 | 10 | .84 |
| 1091 | 30 | 4 | A | 1070 | 10 | .87 |
| 698 | 10 | 2 | C | 1080 | 10 | .92 |
| 788 | 10 | 1 | B* | 1080 | 10 | .93 |
| 791 | 20 | 1 | B* | 1080 | 10 | .93 |
| 980 | 40 | 4 | Other | 1080 | 10 | .91 |
| 1091 | 10 | 3 | A | 1080 | 10 | .87 |
| 1088 | 20 | 3 | A | 1100 | 10 | .82 |

*Short Wavelength Side

Selecting the best wavelength candidates for this four-wavelength model involves a trade-off of competing considerations such as the degree of accuracy that is necessary (e.g., for general population screening tests, a lower accuracy may be acceptable compared to a high level of accuracy needed for measuring and controlling the insulin dosage of a diabetic) and typical instrument design parameters such as product cost (e.g., usability of low-cost IRED's as the light source instead of the high power requirement of a filament-type light bulb). In addition, the minimum risk of encountering an erroneous blood glucose reading is considered as well.

The data in Table III illustrates a number of different regions of the spectrum that provide prime candidate wavelengths for measuring glucose level in the blood. Specifically identified are: "Region A" which covers a spectrum range of approximately 1076 to 1086 nm; "Region B" which covers a spectrum range of approximately 809 to 823 nm; "Region C" which covers a spectrum range of approximately 693 to 701 nm; and "Region D" which covers a spectrum range of approximately 614 to 628 nm.

The above-identified wavelengths represent the midpoint wavelengths or the half-way point between the "A" and "B" wavelengths in Table III. In addition, Table III identifies other candidate mid-point wavelengths which are also practical. These wavelengths are identified as "other" and include: approximately 671 nm, approximately 782 nm, and approximately 1040 nm.

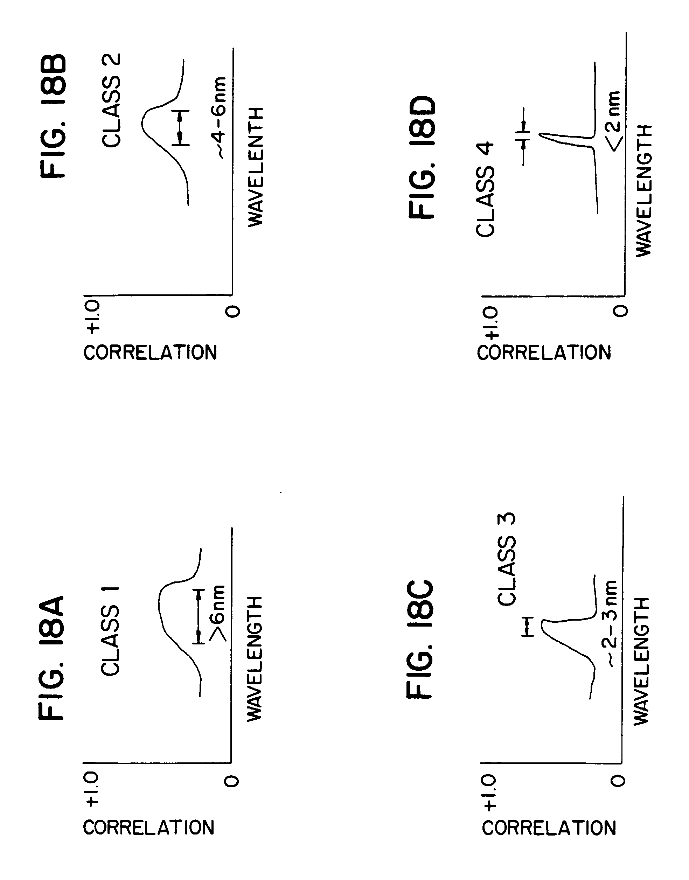

Further, the "class" column corresponds to the shape of the peak of the correlation versus wavelength curve as the "B" value in the numerator is varied. Figures 18A-D illustrate the classes of peaks which correspond to the shape of the correlation versus wavelength curve. Figure 18A illustrates that in "Class 1", the log 1/I values are essentially insensitive to small wavelength errors which represents the ideal situation. Figure 18B shows that the "Class 2" peak allows normal commercial tolerance on center wavelengths of optical filters (plus/minus 2 nanometers). Figure 18C shows that the "Class 3" peak could require tighter than normal commercial tolerances on the center wavelength of the optical filters. A tolerance of approximately +/- 1 nanometer would be required in this class. This tighter tolerance would increase the cost of such optical filters by perhaps a factor of two to four. Figure 18D illustrates that in "Class 4", the measurement is hypersensitive to wavelength errors and would require the optical filter to provide a virtually correct wavelength. A small error would have a significant negative affect on measurement accuracy. The cost of providing such optical filters could be increased by a factor of ten over "Class 3" filters.

Denominator gaps described in Table III (i.e., D-E) are only representative values. As is customary in near-IR quantitative measurement, the denominator gaps can be increased substantially without undue impact on the total correlation coefficient. Thus, for convenience only, the denominator gaps of approximately ten nanometers have been shown in most cases.

In a preferred embodiment, the optimum wavelength candidates shown in Table III are A=771 nm, B=791 nm, D=1070 nm, and E=1080 nm. These wavelength candidates conform to the logical measurement regions defined above for Regions "J" and "K" which remove inaccuracies resulting from water absorption. Furthermore, these wavelengths provide for the highest correlation and allow use of lowest cost IREDs. In addition, these values have "Class 1" characteristics in that they are insensitive to small wavelength errors.

Table IV reproduced below presents the various candidates for the six-wavelength model. The test data was derived in a similar fashion to the test data in Tables I and II. As would be expected, there are a smaller number of candidates for the six-wavelength model compared to the four-wavelength model. This is a direct result of the fundamental difference between the two algorithms. The six-wavelength match uses second derivatives of the optical curve. The second derivative is only of value at the peak absorption points, whereas the first derivative algorithms have twice as many possible candidates since measurement can be made on either side of the peak absorption point.

Table IV

| $d^2OD/d^2OD$ for All Denomination, (D-E: E-F - 10) (R > .8) | | | | | |
|---|---|---|---|---|---|
| Criteria: Must be OK on 3 People, "$R_{min}$" is minimum for lowest person | | | | | |
| DEN "E" | $R_{min}$ | "B" | GAP (A-B)& (B-C) | CLASS | DENOMINATOR GAP |
| 650 | .85 | 635 | 10 | 4 | 10 |
| 650 | .88 | 691 | 20 | 4 | 10 |
| 650 | .86 | 770 | 20 | 3 | 10 |
| 650 | .90 | 791 | 10 | 3 | 10 |
| 650 | .88 | 925 | 10 | 2 | 10 |
| 650 | .84 | 1000 | 20 | 2 | 10 |
| 650 | .86 | 1065 | 10 | 2 | 10 |
| 667 | .91 | 1065 | 40 | 2 | 40 |
| 667 | .90 | 791 | 30 | 2 | 30 |
| 687 | .94 | 825 | 20 | 1 | 50 |
| 820 | .92 | 667 | 40 | 2 | 10 |
| 824 | .94 | 667 | 20 | 2 | 30 |
| 844 | .94 | 667 | 30 | 1 | 10 |
| 883 | .92 | 667 | 30 | 3 | 10 |
| 910 | .87 | 667 | 30 | 4 | 10 |
| 920 | .85 | 656 | 30 | 3 | 10 |
| 920 | .87 | 667 | 40 | 3 | 10 |
| 930 | .90 | 667 | 40 | 2 | 10 |
| 940 | .88 | 667 | 40 | 3 | 10 |
| 950 | .82 | 791 | 30 | 2 | 10 |
| 960 | .90 | 667 | 40 | 3 | 10 |
| 970 | .88 | 667 | 40 | 3 | 10 |

Table IV   (continued)

| d²OD/d²OD for All Denomination, (D-E: E-F - 10) (R > .8) | | | | | |
|---|---|---|---|---|---|
| Criteria: Must be OK on 3 People, "R$_{min}$" is minimum for lowest person | | | | | |
| DEN "E" | R$_{min}$ | "B" | GAP (A-B)& (B-C) | CLASS | DENOMINATOR GAP |
| 980 | .88 | 667 | 40 | 3 | 10 |
| 980 | .85 | 825 | 20 | 3 | 10 |
| 990 | .85 | 667 | 30 | 3 | 10 |
| 1040 | .87 | 667 | 30 | 3 | 10 |
| 1050 | .83 | 667 | 30 | 3 | 10 |
| 1050 | .91 | 825 | 20 | 4 | 10 |
| 1060 | .88 | 667 | 30 | 4 | 10 |
| 1070 | .82 | 667 | 30 | 3 | 10 |
| 1080 | .83 | 788 | 20 | 2 | 10 |

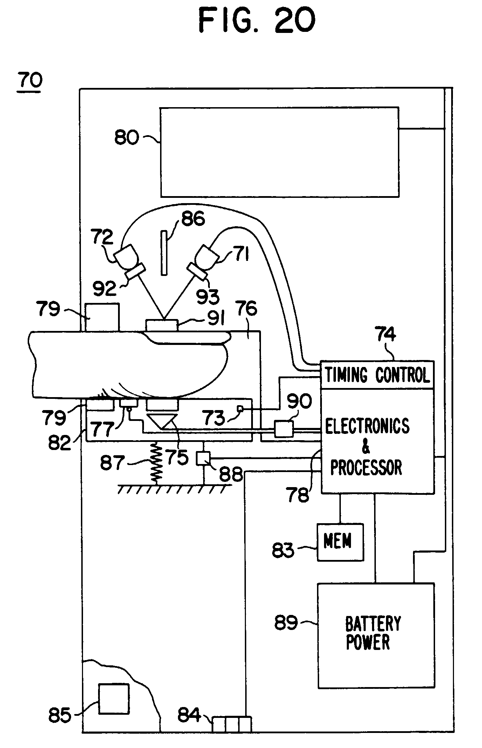

According to another embodiment of the present invention, Figure 20 illustrates a Non-invasive Self Monitoring Glucose Meter (NISMGM) 70 which is designed to measure blood glucose levels using near-IR transmission through the distal portion of the index finger. In this embodiment, the entire analytical instrument, including near-infrared point sources 71 and 72, transmitter, detector 75, amplifier 90, data processing circuitry 78, battery power source unit 89 and readout 80 is contained within a lightweight hand-held unit 70.

As illustrated in FIG. 20, the near-infrared point sources 71 and 72, separated by light baffle 86, are positioned so that near-infrared energy being emitted from the source will be directed through window 91 and onto the skin of the individual. The near-infrared point sources used in a preferred embodiment are IREDs. Further, optical filters 92 and 93 are between the IRED and the window 91 for filtering near-infrared radiation exiting each IRED and thereby allowing a narrow band of near-infrared radiation of predetermined wavelength to pass through.

In a preferred embodiment, the four-wavelength model can be used with the following multiple linear regression algorithm:

$$C = K_o + K_1 [\log 1/I_A - \log 1/I_B]/[\text{Log } 1/I_D$$

$$- \text{Log } 1/I_E] + K_2 T_S + K_3 T_A$$

where $K_o$ through $K_3$ are the calibration constants described above, A, B, C and D are specific wavelengths where the optical data is being measured, $T_S$ is the local surface temperature of the finger in degrees centigrade divided by 100 and $T_A$ is the temperature of the air within the instrument in degrees centigrade divided by 100. Calibration constants $K_2$ and $K_3$ are independent regression terms.

In the embodiment shown in Figure 20, any wavelength candidates may be chosen as described above which yield accurate glucose measurement levels. In a preferred embodiment, the wavelength candidates can be approximately A=771 nm, B=791 nm, C=1070 nm and D=1080 nm. The above wavelengths can be obtained using standard 880 nm IREDs including narrow band pass filters producing wavelengths at 770/790 nm, and standard 950 nm IREDs used at the 1070/1080 nm wavelengths. Also, IREDs having center wavelengths at longer wavelengths than 950 nm could be used as well.

In actual use, it is very important that the fingertip not be exposed to ambient light. Further, it is desirable that the actual measurement be made near the rear of the finger-nail. Figure 20 illustrates a Finger Sizer means 82 to securely position the user's finger inside the instrument and to provide sufficient blockage of ambient light. Spring 87 pushes the Finger Sizer 82 against the bottom of the individual's finger thereby providing a secure fit. Linear potentiometer 88 is connected to Finger Sizer 82 and can measure an individual's finger thickness. In addition, an inflatable diaphragm or foam iris (illustrated at 79) can be used to secure the individual's finger and shield light as well.

Figure 20 further illustrates a measurement reading output device which is a large LCD display 80. Display 80 is advantageously constructed in a large size to facilitate reading by potential users who may have poor eyesight, one of the side effects of diabetes.

In a preferred embodiment, the NISMGM comprises means for displaying blood glucose level in two systems: the mg/d1 for U.S. use and the mmol/L for Canadian use. Processor/controller 78 shown in Figure 20 can perform this function. A change between the two display modes would typically only be required for initial setup. Thus, a slide type switch (not shown) positioned in a non-convenient place (e.g., in the battery compartment) could be used for this function.

Figure 20 shows input/output connector 84 which allows the NISMGM to be connected to a "host instrument" which can determine the calibration constants for the instrument and transfer them automatically to the NISMGM. The input/output connector 84 could also be attached to an external keypad to allow manual entry of calibration constants.

The operation of the NISMGM is described as follows. Prior to making a finger measurement, a front panel push button 85 is pressed which provides for optical standardization which could be the "optical standard" derived from the empty chamber. This measurement would include a "dark correction," which can be a measurement with all IREDs off so that a light leak would be detected and automatically corrected.

Figure 20 illustrates that when the finger is inserted into chamber 76, a built-in thermistor 77 measures the finger's temperature. The temperature measurement will be made at two times, approximately five seconds apart, the actual temperature term $T_S$ being determined by using a logarithmic prediction equation as described in US-A- 4,286,376. Care should be taken that the finger holder 79 fits securely enough around the finger so as to block out light, and yet loose enough that pulse beating does not interfere with the measurement.

A second thermistor 73 is positioned inside the instrument for measuring the ambient temperature. The ambient temperature measurement could be measured at any time prior to the instrument's actual use, but preferably at the same time the optical standard is measured. No logarithmic prediction is needed for the optical measurement. Also, timing control means 74 in control circuitry 78 provides a battery protection feature which shuts off the instrument within approximately two minutes of the last measurement. No off button would be required.

In a further embodiment of the present invention, the NISMGM instrument comprises a memory unit 83 for storing a user's measurement data over a period of time. A built-in clock feature could also store the times at which the measurements were taken. The user would then be able to take the instrument to a doctor who could connect it to an output device, thereby dumping the information. This would allow the doctor to obtain information on the glucose level variability that occurred, for example, during the month.

According to another embodiment of the invention utilizing near-IR transmission analysis techniques, near-IR light energy at bandwidths centering on one or more wavelengths of interest is transmitted through a blood-containing portion of the body of a test subject. The near-IR energy emerges from the test subject, generally opposite from the near-IR source, and is detected by a detector. Following amplification of the detector-generated signal, the amplified output is processed into an output signal indicating the amount of glucose in the subject's blood.

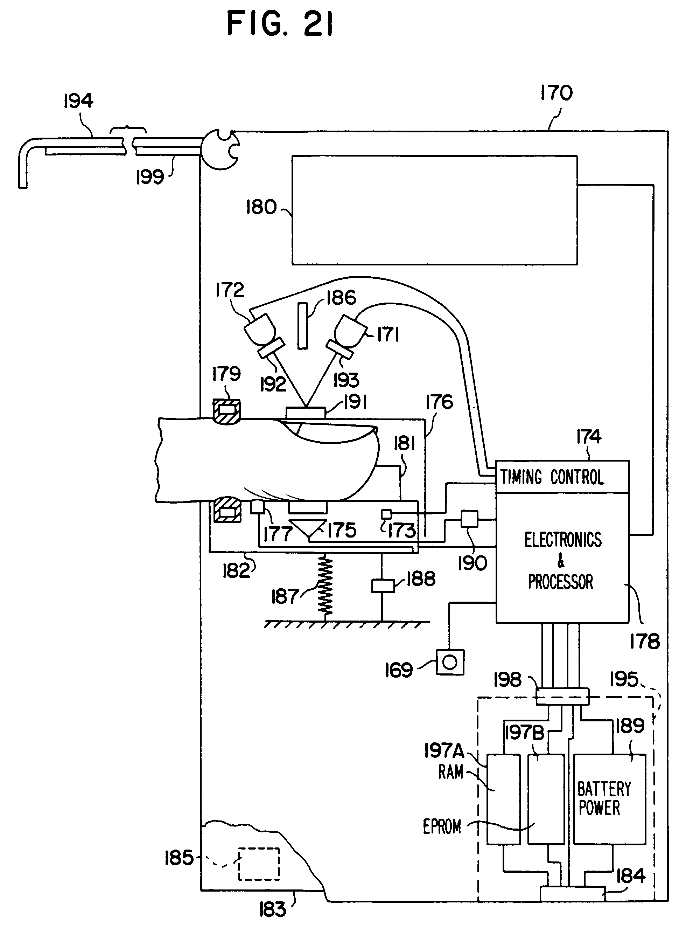

Figure 21 illustrates a Non-invasive Self Monitoring Glucose Meter (NISMGM) 1 which is designed to measure blood glucose levels using near-IR transmission through the distal portion of a test subject's finger. The analytical instrument contains at least one near-infrared energy source for introducing near-infrared energy into the test subject's finger. In one embodiment of the present invention, the introducing means comprises up to six or more near-infrared point sources (near-IR IREDs). IREDs 171 and 172 are shown for illustrative purposes in Figure 21. The analytical instrument also utilizes detector 175 for detecting near-infrared energy emerging from the test subject's body part. Detector 175 is electrically connected to data processing means 178 which, according to its programming, processes the signal produced by the detector 175 into a signal indicative of the quantity of glucose present in the blood of the test subject. Amplifier 190 amplifies the signal produced by the detector 175 before it is received into the processing means 178.

As illustrated in Figure 21, the IREDs 171 and 172, detector 175 and processing means 178 are contained within a lightweight hand-held housing unit 170. In a preferred embodiment, housing unit 170 is machined or molded from plastic.

Illustrative IREDs 171 and 172 are separated by light baffle 186 and are positioned so that the near-IR energy is directed through window 191, which may be light scattering, and onto the skin of the test subject. Optical filters, illustrated at 192 and 193, are positioned between each IRED and the window 191 for filtering the near-IR light, thereby optimizing the band of near-IR light striking the subject.

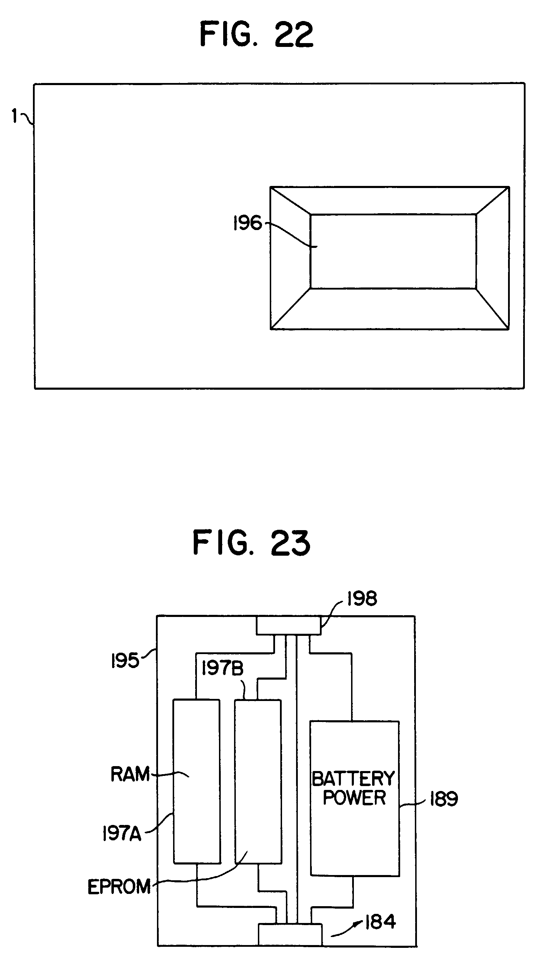

An important feature of the present invention is the cartridge means 195 which engages the housing unit. In a preferred embodiment, the cartridge means 195 is replaceable and is plugged into the analysis instrument 170 through receiving port 196 as illustrated in Figure 22. Cartridge 195 includes electronic memory storage devices 197A and 197B for storing data regarding an individual user and instrument software. By way of example, memory device 197A, such as a RAM, stores a series of glucose readings, the time and the date of all measurements made by the analysis instrument, the total number of measurements made using a particular battery, as well as calibration constants (unique to the individual user) for use by data processor 178. In a preferred embodiment, the memory device 197B is an EPROM-type chip that contains the software for operating the analytical instrument 170, thus facilitating reprogramming of instrument 170 by inserting an updated cartridge.

In certain embodiments, cartridge 195 also comprises a power source means, such as the illustrated battery 189, for supplying power to the near-infrared quantitative analysis instrument. Memory devices 197A and 197B and battery 189 are in electrical communication with the processing means 178 via interface 198.

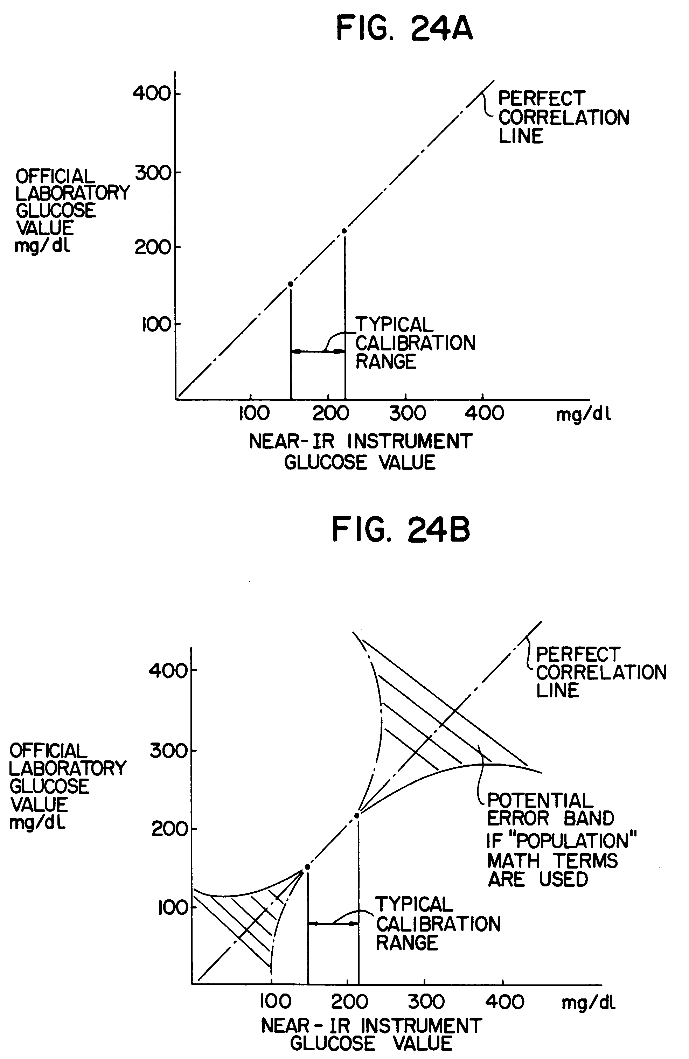

An instrument for non-invasive measurement of blood glucose should provide accurate readings for any individual's glucose level which may be anywhere from about 40 mg/dl to as high as 500 mg/dl. In a typical glucose tolerance test used for calibration, changes in an individual's glucose level can be expected within the range of approximately 50 mg/dl to approximately 150 mg/dl. As a result, providing a custom calibration for an instrument covering the entire range is essentially impossible. Extrapolating information from this small calibration data using the single variable term algorithms shown above can cause errors when measuring glucose levels in the extreme regions of the entire range of possible glucose levels. These potential errors result from the non-linearity of extrapolation values as illustrated in Figures 24A and 24B.

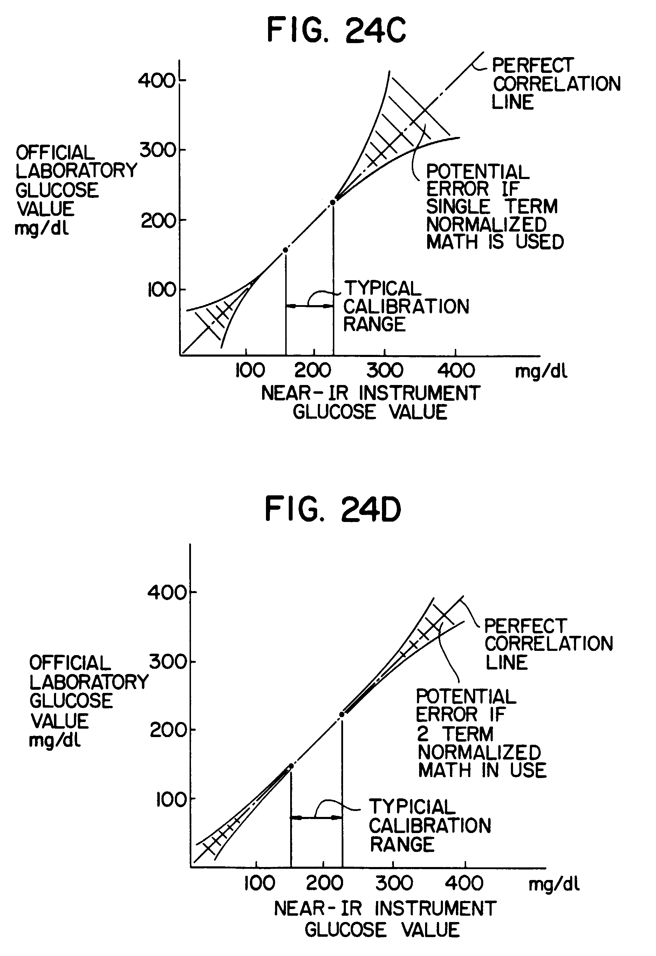

To correct for nonlinear extrapolation, algorithms comprising multiple regression terms are used which enable accurate prediction of blood glucose measurement over a wide range. Such terms can be individual log 1/l terms or can be a multiple number of first or second derivative terms with or without a normalizing denominator. Extrapolation is most reliable, however, using normalized division terms which are self-correcting for many types of measurement variability, as illustrated in Figures 24C and 24D. This self-correction occurs because the numerator and denominator normally are multiplied by the same error, thereby self-cancelling the error. Examples of these terms include normalized first and second derivative division terms. In one embodiment, algorithms containing two or more of the normalized division terms are used.

Data on a plurality of physical parameters of the test subject can also be utilized in conjunction with multiple wavelength near-infrared analysis, as in prior US-A-4,633,087, to improve the accuracy of the present blood glucose measurements. Further, blood glucose measurement accuracy can be improved by eliminating error which may result from bias shift in the near-infrared sources.

Each replaceable cartridge 195 is identified as belonging to a specific individual, because it contains custom calibration values for that individual. A user protection means is provided to insure that a replacement cartridge containing an individual's calibration information is used only by that individual, in the proper unit. In one embodiment, user protection is accomplished by a "handshake" code transmitted between the replacement cartridge and the unit. The encoding procedure comprises copying one or more calibration constant in the cartridge into the unit, which constant is used as the "handshake." In a preferred embodiment, all calibration constants for a particular individual are used as the "handshake."

In the embodiment shown in Figure 21, wavelengths for conducting the near-IR analysis are chosen so as to yield accurate glucose measurement levels. In one embodiment, the wavelengths are approximately A=771 nm, B=791 nm, C=1070 nm and D=1080 nm. The 771 nm and 791 wavelengths can be obtained using standard 880 nm center wavelength IREDs in combination with narrow bandpass filters. IREDs having center wavelengths at longer wavelengths, such as the standard 950 nm IRED, are useful for producing 1070 nm and 1080 nm output when combined with appropriate optical filters. In a preferred embodiment, the wavelengths are approximately A=667 nm, B=687 nm, D=1059 nm and E=1079 nm.

It is very important that the test subject's fingertip not be exposed to ambient light. Further, it is desirable that the actual measurement be made near the rear of the finger-nail. Finger stop 181 illustrated in Figure 21 facilitates properly positioning the test subject's finger. Figure 21 also illustrates a finger retainer 182 to securely position the user's finger inside the instrument and to provide sufficient blockage of ambient light. Spring 187 pushes the finger retainer 182 against the bottom of the test subject's finger thereby providing a secure fit. Linear potentiometer 188 is connected to finger retainer 182 and can measure an individual's finger thickness. In addition, an inflatable diaphragm or rubber/foam iris (illustrated at 179) is used to secure the test subject's finger and shield light. The inflatable diaphragm can measure the blood pulse as well.

In a preferred embodiment, light shielding cover 194, shown in Figure 21, is rotatably attached to the analytical instrument 170 and closes over the finger chamber 176 providing essentially total blockage of ambient light. Cover 194 extends along the entire side of the analytical instrument and is securable at the base thereof in recess 183. Rubber strip 199 is secured to cover 194 and further facilitates blocking ambient light.

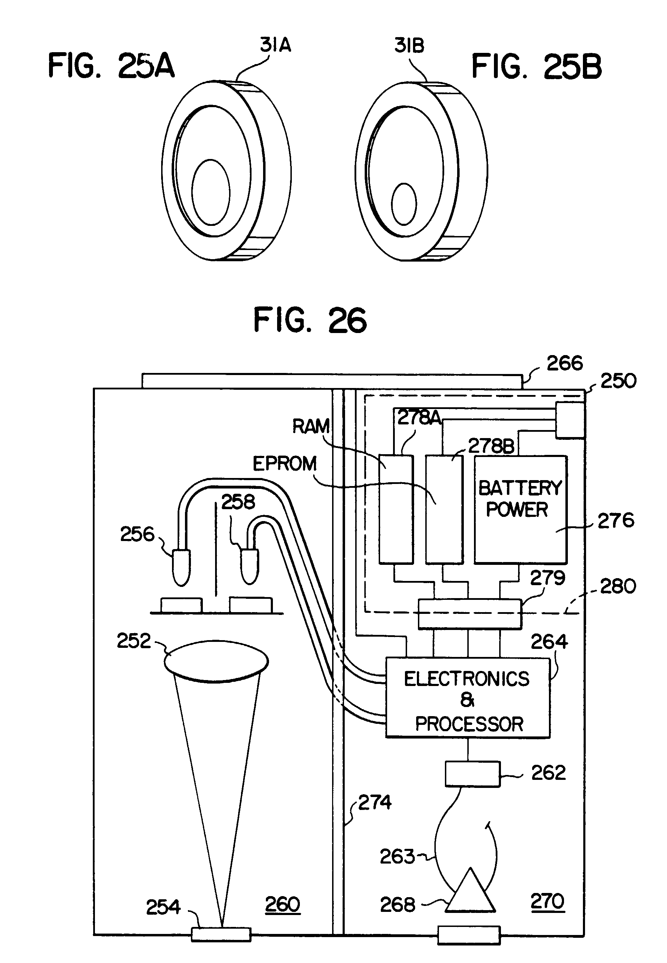

To facilitate the instrument's use by both adults and children, the finger measurement sections of the instrument allow for two or more different sizes of inserts. As illustrated in Figures 25A and 25B, insert 31A is suitable for the size of adult fingers and insert 31B is suitable for the size of children's fingers. Inserts accommodating a finger of any size can be used.

Figure 21 further illustrates an LCD display 180. Display 180 is advantageously constructed in a large size to facilitate reading by potential users who may have poor eyesight, one of the side effects of diabetes.

In a preferred embodiment, the analytical instrument comprises means for displaying blood glucose level in two systems: the mg/dl unit for U.S. use and the mmol/L unit for Canadian use. Processor means 178 shown in Figure 21 can perform this function. A change between the two display modes would typically be made during initial setup. Thus, a slide-type switch (not shown) positioned in a non-convenient place (e.g., in the battery compartment) could be used to select the desired display.

Figure 23 shows input/output connector 184 which forms part of the replaceable cartridge 195 and is electrically connected to controller/processor 178 of the analytical instrument 170. Input/output connector 184 allows the analytical instrument 170 to be connected to a "host instrument" which enables calibration constants determined by the host instrument, as described above, to be automatically transferred to the analytical instrument during calibration setup. In addition, the input/output connector 184 can be attached to an external keypad which allows manual entry of calibration constants.

The operation of the non-infrared quantitative analysis instrument will be readily apparent. Prior to making a finger measurement, a front panel push button 185 is pressed which provides for optical standardization. Optical standardization is accomplished by measuring the empty finger chamber. Standardization also can include a "dark correction" measurement with all IREDs off so that a light leak is detected and automatically corrected for.

Figure 21 illustrates that when the finger is inserted into chamber 176, a built-in thermistor 177 measures the finger's temperature. The temperature measurement will be made at two times, approximately five seconds apart, with a temperature reading being determined by using a logarithmic prediction equation as described in US-A-4,286,376. Care should be taken to ensure that the finger holder 182 fits securely enough around the finger so as to block out light, and yet loosely enough to ensure that pulse beating does not interfere with the measurement. Better precision can be achieved if a single measurement involves inserting the finger twice.

A second thermistor 173 is positioned inside the analytical instrument 170 for measuring the ambient air temperature therein. The ambient air temperature measurement could be made at any time prior to the instrument's actual use, but preferably at the time the optical standard is measured. No logarithmic prediction is needed for the instrument air temperature measurement. Also, timing control means 174 in processor means 178 provides a battery protection feature which shuts off the instrument within a predetermined period of time after the a measurement is taken. In a preferred embodiment of the present invention, the battery protection feature shuts off the instrument within approximately two minutes of the last measurement. Although one may additionally be provided, this protection feature would alleviate the need for an "Off" button.

Processor means 178 and memory devices 197A and 197B advantageously enable a user to recall his or her previous glucose readings, including the time and date of those readings. In a preferred embodiment, between approximately eight and ten prior readings can be recalled in reverse order.

The analytical instrument preferably comprises means for indicating on display 180 the number of days remaining before the replaceable cartridge must be changed. This information can be accessed from memory device 197A at any time by pressing push button 185. In a preferred embodiment, this information will automatically appear on display 180 when the instrument is turned on, beginning five days prior to replacement. The analytical instrument 170 also includes a battery protection feature which prevents excessive battery use. During the calibration process, many battery-draining optical scans are required. In a preferred embodiment, pre-programmed circuitry permits a user to make no more than five successive readings without waiting a minimum of ten minutes before taking further readings.

In other versions, the analytical instrument utilizes a speech synthesizer 169 electrically connected to processor means 178 for providing instrument speech capability. This feature is used to provide patient prompting as well as providing an audio readout. This is of particular value to patients whose eyesight is excessively poor.

In another aspect of the present invention, a lightweight hand-held instrument uses the principle of light interactance to measure blood glucose level non-invasively by locating an optical transmitter and a detector on the skin surface near either an artery or vein.

In a lightweight, hand-held interactance analysis instrument 250 illustrated in Figure 26, included is one or more means for providing at least one point source of near-infrared energy of a predetermined bandwidth of interest which is positioned within a first chamber 260 of the instrument 250. The near-infrared point source means is positioned so that near-infrared energy being emitted from the point source means will be focused by lens 252 through window 254 and onto the skin of the test subject. The near-infrared point source means may comprise one or a plurality of infrared emitting diodes (IREDs). Two such IREDs 256 and 258 are illustrated in Figure 26. In other embodiments employing a plurality of IREDs, three, four or more IREDs may be utilized as the point source means.

An optical detector, illustrated schematically in Figure 26 and designated by reference numeral 268, is disposed within a lower end portion of second chamber 270. Inner wall 274 is positioned between detector 268 and illumination section 260, thereby providing an optically-isolated mask which prevents near infrared radiation from the point source means and/or lens 252 from impinging directly on detector 268. The near-infrared optical detector 268 generates an electrical signal when near-infrared radiation is detected thereby.

The optical detector 268 is connected to the input of an electrical signal amplifier 262 by suitable electrical conducting means 263. Amplifier 262 may be an inexpensive integrated circuit (IC) signal amplifier, and amplifies the signals generated when near-IR energy strikes detector 268. The output of amplifier 262 is fed to a controller/data processor and display driver 264 which provides a signal to readout device 266.

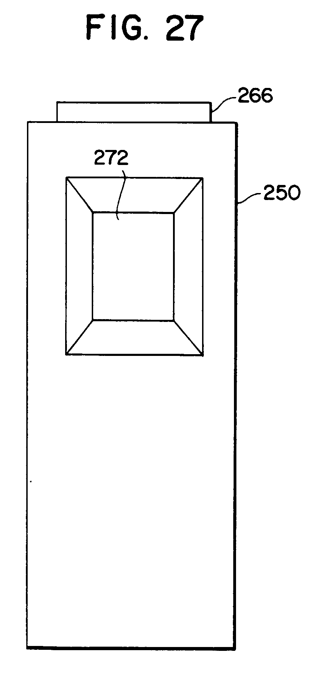

Interactance analysis instrument 250 further includes a replaceable cartridge means 280 which engages the housing unit. In a preferred embodiment, the cartridge means 280 is plugged into the analysis instrument 250 through receiving port 272 as illustrated in Figure 27. Cartridge 280 is substantially identical to cartridge 195 discussed above and includes a battery power source 276, an electronic memory storage device 278A, such as a RAM, for storing date regarding an individual user and memory storage device 278B, such as an EPROM, for storing the instrument's software. In addition, cartridge 280 performs the same operational functions as disclosed with reference to cartridge 195. Memory devices 278A and 278B and battery 279 are in electrical communication with controller/data processor 264 via interface 279.

In use, the analysis instrument 250 is positioned so that its flat bottom surface rests on the skin directly above a prominent vein of the wrist of the test subject. Interactance instrument 250 is particularly useful for measuring the blood glucose level of small children whose fingers may be too small to achieve accurate results using the analysis instrument of Figure 21.

In another aspect of the present invention, a method for custom calibrating the near-infrared analysis instrument for the individual user is disclosed. In conventional near-infrared analysis, the calibration procedure requires a significant number of "samples" (i.e., a set of optical measurements) with known laboratory analysis of the parameter of interest (e.g., protein in wheat). The need for individual laboratory analyzed samples in conventional analysis results from the fact that each sample, and accordingly the parameter measurement, is totally independent of the previous sample. For example, when calibrating an instrument to measure protein in wheat, each wheat sample requires separate laboratory analysis.

However, in custom calibration for blood glucose, that *a priori* assumption is not true. It is known that if a diabetic eats food, a diabetic's blood glucose level will increase over the next fifteen minutes to a half an hour. This known characteristic of the direction of change in an individual's blood glucose level is an important factor for simplified custom calibration.

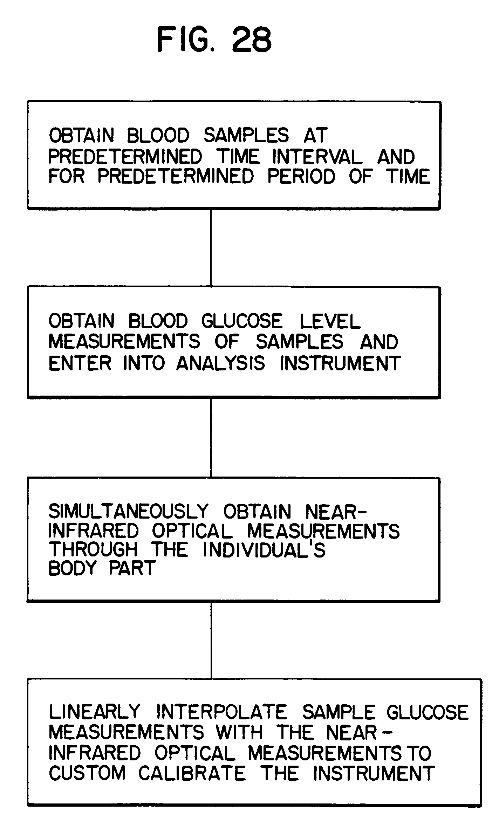

Figure 28 illustrates an easy and reliable method for custom calibration wherein a plurality of blood samples are obtained from an individual at predetermined time intervals, which do not need to be of equal duration, and for a predetermined period of time. Blood glucose levels are then obtained for each blood sample by any suitable reference method such as a Glucose Meter 2300G, manufactured by Yellow Springs Instrument Co. This information will serve as the laboratory data. The blood samples and blood glucose level measurements can be obtained using any other suitable means, such as a commercially available Biostator.

In a preferred embodiment, the blood glucose level measurements are obtained from a widely available Self-Monitoring Glucose Meter ("SMGM"). The technique employed in SMGM's involves using a lancet to draw blood from a finger-tip. A drop of blood is then placed on a chemically loaded plastic strip which is inserted into the instrument. The instrument measures and then digitally displays the blood glucose levels.

The predetermined time period in which the blood samples are taken is typically approximately once every 10-20 minutes and for approximately one-half hour to an hour and a half. The blood glucose level data is then entered into any suitable computation instrument, such as a computer, which can perform multiple regression analysis. In a preferred embodiment, the blood glucose level data is entered into the near-infrared analysis instrument.

Also in a preferred embodiment, the predetermined time interval is selected so as to obtain actual blood glucose measurements at different levels while the individual's blood is being "spiked" (i.e., caloric ingestion). For example, a first blood glucose reading is taken after an overnight fast. Following this reading, the individual will either take and ingest a dextrose solution or eat a meal which will elevate the individual's blood glucose level. An additional finger poke measurement is then made after approximately fifteen minutes. This is repeated approximately every fifteen minutes for one hour. A total of four finger poke measurements are taken, in the preferred embodiment. The sample blood glucose level measurements could also be taken at predetermined time intervals which are not of equal duration, for example, after ten minutes, twenty-five minutes, forty-five minutes and after one hour and ten minutes. The predetermined time interval can be any time interval which will enable the individual's blood glucose level to be measured during at least a period where the individual's blood glucose level changes.

During the time period in which the blood samples are taken, near-infrared optical measurements are made and recorded using the non-invasive blood glucose instrument. These optical measurements should be made approximately once per minute. A total of approximately 60 sets of optical information will be recorded in the preferred embodiment.

The method of the present invention is utilized to calibrate a near-infrared analysis instrument not only for blood glucose levels, but for any known characteristic of the blood. For example, *a priori* knowledge of insulin level characteristics in the body is known and the method of the present invention could be used to calibrate a near-infrared instrument for measurement of insulin levels.

The calibration regression analysis performed on the optical measurements will be described hereinafter. The calibration regression analysis of the present invention utilizes means for linearly interpolating the blood glucose level measurements made from the individual blood samples with the near-infrared optical measurements. Specifically, linear interpolation is used to assign laboratory values to the near-infrared optical measurements, between adjacent pair of the four finger poke glucose level measurements, which are taken at approximately fifteen minutes apart in the preferred embodiment. The linear interpolation is performed to provide glucose values for the specific times that the optical measurements are made, thus becoming the independent variable of the regression analysis. Thus, in the preferred embodiment, a set of four actual finger poke measurements will provide information for approximately 60 sets of the near-infrared optical measurements. The regression analysis is performed by a signal processor in any suitable method, such as disclosed above.

Since linear interpolation involves mathematically assigning expected values based on known values using an interpolation algorithm, the potential for error exists. In near-infrared analysis, it is known that the laboratory value assigned at any particular time by the interpolation process will potentially be in error from the actual blood glucose value at that moment. These errors typically result from the inaccuracy of the SMGM readings and from errors due to using interpolated values between the times of SMGM readings.

Although potential for error in the interpolated values exists, considerable experimentation in near-infrared technology has shown that such errors do not significantly affect calibration accuracy. The reason that accuracy is not significantly sacrificed is that the number of calibration samples, i.e., near-infrared optical measurements, is much larger than the number of regression terms in the regression algorithm. For example, the regression analysis performed in the present invention, as disclosed above, can use approximately three regression terms. Typically, acceptable accuracy occurs in near-infrared calibration where approximately ten samples are used for each regression term. In a preferred embodiment, approximately 60 samples are used and the total number of regression terms is approximately three or four. Thus, approximately fifteen samples per regression term are used. Moreover, this procedure can be repeated over several days which will enhance the statistical significance.

A near-infrared noninvasive blood glucose measurement instrument which can be custom calibrated employing the method of the present invention is illustrated schematically in Figure 21, as disclosed above.

In a preferred embodiment, the need to provide a data link to a host computer is eliminated by enabling the individual sample blood glucose level measurements to be entered into the analytical instrument through a keyboard on the instrument itself (not shown).

The method for custom calibrating a non-invasive blood glucose measurement instrument according to the present invention can also be used to calibrate analytical instruments which utilize near-infrared reflectance or interactance, such as disclosed above.

This novel method overcomes the inconveniences of the prior art methods by providing easy and reliable custom calibration of non-invasive blood glucose measurement instruments which can readily be done on an at-home basis.

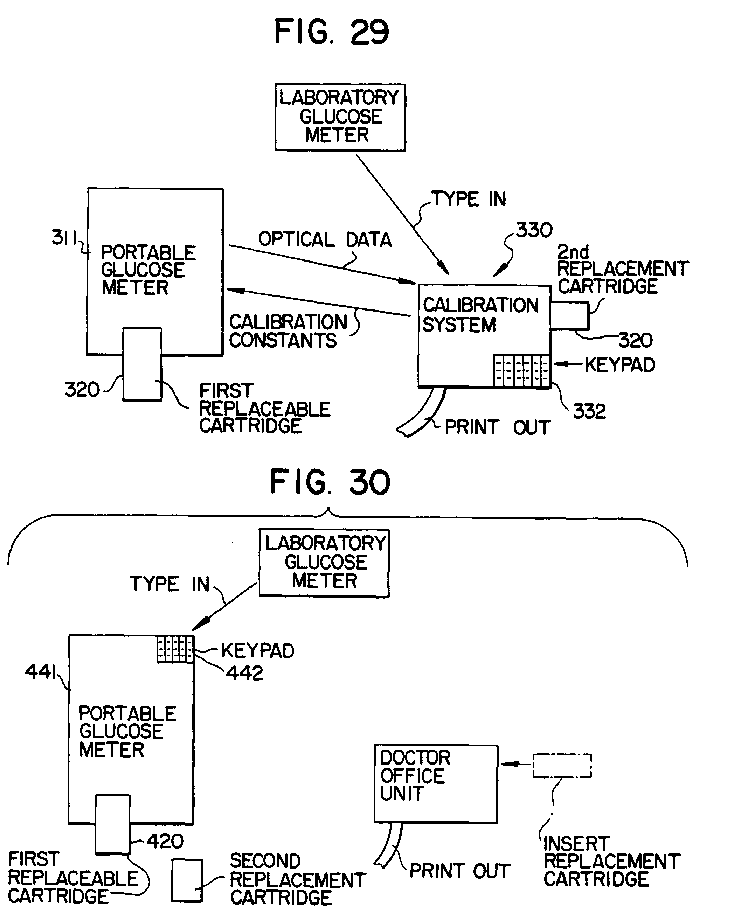

Additional embodiments of near-infrared non-invasive blood glucose measurement instruments which can be custom calibrated according to the method of the present invention will be described with reference to Figures 29 and 30.

The near-infrared analytical instruments 311 and 441, illustrated in Figures 29 and 30 respectively, operate substantially identically as disclosed with reference to Figure 21 above. The custom calibration procedure will be described in two alternate methods. First, as illustrated in Figure 29, a calibration system 330 is connected to the analytical instrument 311 and collects both optical data as measurements are taken and the time of each measurement. The calibration system 330 also allows the glucose values from the finger poke measurements to be hand-entered via keypad 332 on the calibration system 330 at approximately 15-minute intervals for up to 1.5 hours. When sufficient data has been received, such as after the above-described predetermined time intervals, the calibration system performs a multiple regression analysis, calculating the calibration constants for the linear regression algorithms. The calculated calibration constants are then transferred to the replaceable cartridges 320 plugged into the calibration system 330, or into a replaceable cartridge installed in the analytical instrument 311, and another inserted into the calibration system 330. In this approach, the calibration system 330 could be in a doctor's office and could also provide a printout device for the replaceable cartridges.

An alternate approach, illustrated in Figure 30, does not require a calibration system as discussed above. In this approach, the replaceable cartridge contains EPROMs rogrammed to perform multiple regression analysis. The analytical instrument 441 has a key pad 442 having, typically, 11 keys (e.g. "0" through "9" plus an ENTER key) to allow the finger poke readings to be entered. In another embodiment, the finger poke readings are entered from an external keyboard or a replaceable cartridge. After the regression analysis is performed, the analytical instrument stores the calibration constants in the replaceable cartridge. At the same time, the analytical instrument erases the regression program allowing the EPROM to be used to store time/data and glucose values for a predetermined period of time, e.g., three months.

In another aspect of the invention, a method is disclosed for providing custom calibration which is valid over a range of skin temperatures. It is well established that temperature affects the energy absorption in near-infrared water band. Further, since the human body does not maintain constant temperatures at the extremities, i.e., fingers, calibration accuracy can be improved by including the effects of temperature variation.

The effect of temperature variations in blood glucose calibration has a direct applicable analogy in near-infrared (NIR) agricultural instruments. For example, if calibration for measurement of protein in wheat is performed with all wheat samples near room temperature, large errors would occur if that calibration is then used to predict the protein in wheat samples at significantly different temperatures.

This potential error is largely avoided by deliberately changing the temperature of samples during wheat calibrations. For example, some wheat samples are stored in a refrigerator and then immediately measured on an NIR instrument. Other wheat samples are stored in freezers and then immediately measured on the NIR instrument. This approach has proven to be very successful for agricultural products, such as wheat, and has proven to provide accurate measurements at extreme temperatures.

An analogous approach provides accurate blood glucose calibration. Specifically, an individual's finger temperature is forced to have a range during the calibration process. Optical recordings are taken during the calibration at a range of skin temperatures.

Finger temperature variations can be induced using any conventional approach. For example, according to one technique of the present invention a small vessel is filled with water at room temperature. The individual to be tested then inserts the hand into a plastic bag leaving the top edge of the bag open. The bagged hand is then inserted into the water avoiding water contact with the skin. The hand is left in the water for at least one minute. Good heat transfer will result as the water pressure will force the air out of the plastic bag thereby snugly pressing the plastic against the hand. The hand is then removed and the near-infrared measurement is performed. The above-described steps are then repeated using water having a different temperature, i.e., approximately 38 to 49°C (100 to 120°F). Although any number of different temperature measurements could be used, the above approach allows the calibration to be performed with three different temperatures, including a measurement with no water immersion.

A test was performed to illustrate the effect of temperature variation on optical absorption wherein separate near-infrared measurements were taken for three finger temperature variations: (1) Hand in plastic bag immersed for 60 seconds in cool tap water (13°C)(55°F); (2) Normal skin temperature (no water immersion); and (3) Hand in plastic bag immersed for 60 seconds in warm water (62°C)(103°F).

TABLE V

| | Measured Finger Temperature | Peak Optical Absorption | Rate of Shift |
|---|---|---|---|
| Cool tap Water (13°C) (55°F) | 30.8°C | 972.3 nm | $\dfrac{972.3 - 971.8}{30.8 - 34.3}$ |
| No Water Immersion | 32.9°C | 972.0 nm | |
| Warm Tap Water (62°C) (103°F) | 34.3°C | 971.8 nm | $= -0.14 \ nm \ /°C$ |

The test results set out in Table V above illustrate that the peak optical absorption shifts with finger temperature at a rate of approximately -0.14 nm/°C.

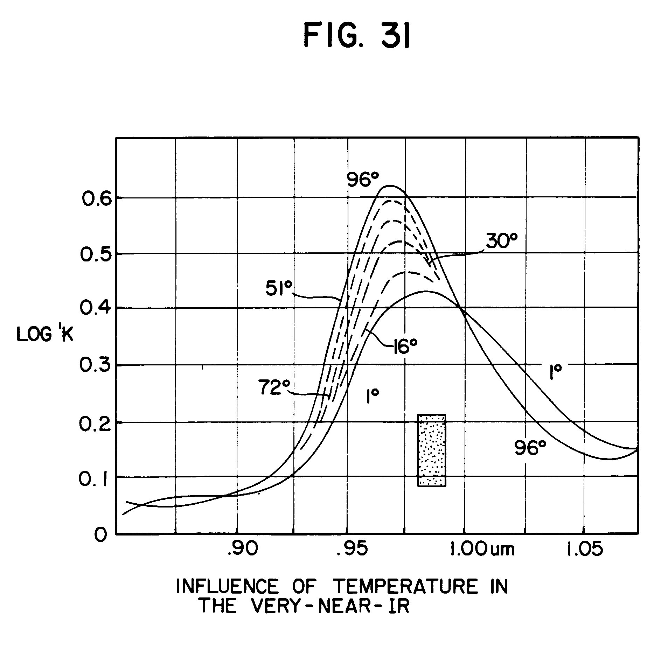

The rate of wavelength shift resulting from finger temperature variation is compared with the rate of wavelength shift caused by temperature variations in water. The magnitude of the wavelength shift can be calculated from the data in Figure 31.

$$\text{Peak wavelength at } 11°C = 980 \ nm$$

$$\text{Peak wavelength at } 96°C = 970 \ nm$$

$$\text{Rate Of Shift} = 980\text{-}970/1\text{-}96 = -0.11/°C$$

The test results indicate that the measured rate of shift is essentially identical for the finger measurement as it is for pure water. This illustrates that by imposing a temperature variation in the glucose calibration, the calibration will have greater validity over all finger temperatures.

Although the invention has been described in connection with certain preferred embodiments, it is not limited to them. Modifications within the scope of the following claims will be apparent to those skilled in the art. For example, accurate measurements can be obtained from parts of the body besides the wrist and the finger. The algorithm used to calculate blood constituent concentration(s) can be altered in accordance with known near-infrared analytical techniques.

## Claims

1. A near-infrared quantitative analysis instrument (10) for non-invasive measurement of blood glucose in blood present in a body part of a subject, comprising:

(a) introducing means including a near infrared energy source (16,16') for introducing near-infrared energy into blood present in a body part of a subject;

(b) detecting means (28) for detecting near-infrared energy emerging from the body part;

(c) positioning means (20) for positioning both the near-infrared introducing means (16,16') and the detecting means (28) closely adjacent to the body part; and

(d) processing means (34) for processing a first electrical signal produced by the detecting means (28) into a second signal indicative of the quantity of glucose present in the blood of the subject, characterised in that the processing means processes the first signal according to a formula including a temperature term $K_2T_S$ wherein $T_S$ represents the local surface temperature of said body part and $K_2$ is a calibration constant and wherein the formula is one of

$$C = K_0 + K_1 [\log 1/I_A - \log 1/I_B] + K_2T_S \qquad\qquad (i)$$

wherein C is concentration of glucose present in the blood, $K_0$ is an intercept constant, $K_1$ is the line slope of

$$[\log 1/I_A - \log 1/I_B],$$

and $\log 1/I_A$ and $\log 1/I_B$ each represent an optical density value at corresponding wavelengths A and B;

$$C = K_0 + K_1 [\log 1/I_A - 2*\log 1/I_B + \log 1/I_C] + K_2T_S \qquad\qquad (ii)$$

wherein $K_1$ is the line slope of

$$[\log 1/I_A - 2*\log 1/I_B + 1/I_C],$$

$\log 1/I_C$ represents an optical density value at wavelength C, and C, $K_0$, $\log 1/I_A$ and $\log 1/I_B$ are as defined in formula (i);

$$C = K_0 + K_1 \frac{[\log 1/I_A - \log 1/I_B]}{[\log 1/I_D - \log 1/I_E]} + K_2T_S \qquad\qquad (iii)$$

wherein $K_1$ is the line of slope of

$$\frac{[\log 1/I_A - \log 1/I_B]}{[\log 1/I_D - \log 1/I_E]},$$

$\log I/I_D$ and $\log 1/I_E$ each represent an optical density value at corresponding wavelengths D and E, and C, $K_0$, $\log 1/I_A$ and $\log 1/I_B$ are as defined in formula (i); or

$$C = K_0 + K_1 \frac{[\log 1/I_A - 2*\log 1/I_B + \log 1/I_C]}{[\log 1/I_D - 2*\log 1/I_E + \log 1/I_F]} + K_2T_S \qquad\qquad (iv)$$

wherein $K_1$ is the line slope of

$$\frac{[\log 1/I_A - 2*\log 1/I_B + \log 1/I_C]}{[\log 1/I_D - 2*\log 1/I_E + \log 1/I_F]},$$

$\log 1/I_C$, $\log 1/I_D$, $\log 1/I_E$ and $\log 1/I_F$ each represent an optical density value at corresponding wavelengths C, D, E and F, and C, $K_0$, $\log 1/I_A$ and $\log 1/I_B$ are as defined in formula (i).

**2.** The analysis instrument of claim 1 wherein said energy source comprises a plurality of near-infrared emitting diodes (16,16').

**3.** The analysis instrument of claim 2 wherein said plurality of near-infrared emitting diodes (16,16') comprises six near-infrared emitting diodes.

**4.** The analysis instrument of any one of claims 1 to 3 wherein optical filters (123) corresponding to the optimum wavelength values for A and B for a given subject are installed in said instrument (10).

**5.** A non-invasive method for quantitatively analysing blood glucose in blood of a subject, comprising:

(a) introducing at least one pair of wavelengths of near-infrared energy from a near-infrared energy source (16,16') into blood within a body part of the subject, said pair of wavelengths being within the range of about 600 to about 1100 nanometers;
(b) detecting near-infrared energy emerging from the subject with a detector (28) which provides a first signal upon detecting energy emerging from the subject; and
(c) processing the first signal to provide a second signal indicative of the amount of glucose present in the body of the subject,

characterised in that
the first signal is processed according to a formula including a temperature term $K_2 T_S$ wherein $T_S$ represents the local surface temperature of said body part and $K_2$ is a calibration constant and wherein the formula is one of

$$C = K_0 + K_1 [\log 1/I_A - \log 1/I_B] + K_2 T_S \tag{i}$$

wherein C is concentration of glucose present in the blood, $K_0$ is an intercept constant, $K_1$ is the line slope of

$$[\log 1/I_A - \log 1/I_B],$$

and $\log 1/I_A$ and $\log 1/I_B$ each represent an optical density value at corresponding wavelengths A and B;

$$C = K_0 + K_1 [\log 1/I_A - 2*\log 1/I_B + \log 1/I_C] + K_2 T_S \tag{ii}$$

wherein $K_1$ is the line slope of

$$[\log 1/I_A - 2*\log 1/I_B + 1/I_C],$$

$\log 1/I_C$ represents an optical density value at wavelength C, and C, $K_0$ and $\log 1/I_A$ and $\log 1/I_B$ are as defined in formula (i);

$$C = K_0 + K_1 \frac{[\log 1/I_A - \log 1/I_B]}{[\log 1/I_D - \log 1/I_E]} + K_2 T_S \tag{iii}$$

wherein $K_1$ is the line of slope of

$$\frac{[\log 1/I_A - \log 1/I_B]}{[\log 1/I_D - \log 1/I_E]},$$

$\log 1/I_D$ and $\log 1/I_E$ each represent an optical density value at corresponding wavelengths D and E, and C, $K_0$, $\log 1/I_A$ and $\log 1/I_B$ are as defined in formula (i);

$$C = K_0 + K_1 \frac{[\log 1/I_A - 2*\log 1/I_B + \log 1/I_C]}{[\log 1/I_D - 2*\log 1/I_E + \log 1/I_F]} + K_2 T_S \qquad \text{(iv)}$$

wherein $K_1$ is the line slope of

$$\frac{[\log 1/I_A - 2*\log 1/I_B + \log 1/I_C]}{[\log 1/I_D - 2*\log 1/I_E + \log 1/I_F]} ,$$

$\log 1/I_C$, $\log 1/I_D$, $\log 1/I_E$ and $\log 1/I_F$ each represent an optical density value at corresponding wavelengths C, D, E and F, and C, $K_0$, $\log 1/I_A$ and $\log 1/I_B$ are as defined in formula (i); or

$$C = K_0 + K_1 \frac{[\log 1/I_A - \log 1/I_B]}{[\log 1/I_D - \log 1/I_E]} + K_2 T_S + K_3 T_A \qquad \text{(v)}$$

wherein $K_1$ is the line of slope of

$$\frac{[\log 1/I_A - \log 1/I_B]}{[\log 1/I_D - \log 1/I_E]}$$

$K_3$ is a calibration constant, $\log 1/I_D$ and $\log 1/I_E$ each represent an optical density value at corresponding wavelengths D and E, $T_A$ represents the ambient temperature of said instrument, and C, $K_0$, $\log 1/I_A$ and $\log 1/I_B$ are as defined in formula (i).

6. The method of claim 5 wherein said at least one pair of wavelengths is individually selected for a subject by the following:

(a) sequentially introducing near-infrared energy into said body part of a subject at a plurality of center wavelengths, said plurality of center wavelengths being separated by a constant wavelength;
(b) measuring a glucose level of said subject at a time of low glucose level and measuring a glucose level of said subject at a time of higher glucose level; and
(c) determining from the measuring step which center wavelengths provide the maximum difference and using the determined wavelengths in subsequent measurements for said subject.

**Patentansprüche**

1. Infrarot-C-Gerät (10) für die quantitative Analyse zur nicht-invasiven Messung von Blutglukose im Blut, das in einem Körperteil eines Untersuchten vorhanden ist, das umfaßt:

a) eine Einleiteinrichtung einschließlich einer Quelle (16,16') von Infrarot-C-Energie, die Infrarot-C-Energie in Blut einleitet, das in einem Körperteil eines Untersuchten vorhanden ist;

b) eine Erfassungseinrichtung (28), die Infrarot-C-Energie erfaßt, die aus dem Körperteil austritt;

c) eine Positioniereinrichtung (20), mit der sowohl die Infrarot-C-Einleiteinrichtung (16,16') als auch die Erfassungseinrichtung (28) nahe an den Körperteil angrenzend positioniert werden; und

d) eine Verarbeitungseinrichtung (34), die ein erstes elektrisches Signal, das durch die Erfassungseinrichtung (28) erzeugt wird, zu einem zweiten Signal verarbeitet, das die Menge an Glukose anzeigt, die in dem Blut des Untersuchten vorhanden ist, **dadurch gekennzeichnet**, daß die Verarbeitungseinrichtung das erste Signal entsprechend einer Formel verarbeitet, die einen Temperaturterm $K_2 T_S$ enthält, wobei $T_S$ für die lokale Oberflächentemperatur des Körperteils steht und $K_2$ eine Eichkonstante ist, und wobei es sich bei der Formel um eine der folgenden handelt:

$$C = K_0 + K_1 \, [\log 1/I_A - \log 1/I_B] + K_2 T_S \tag{1}$$

wobei C die Konzentration von Glukose ist, die in dem Blut vorhanden ist, $K_0$ eine Achsenabschnittskonstante ist, $K_1$ die Gefällinie von

$$[\log 1/I_A - \log 1/I_B]$$

ist und $\log 1/I_A$ und $\log 1/I_B$ jeweils einen Wert der optischen Dichte bei entsprechenden Wellenlängen A und B darstellen;

$$C = K_0 + K_1 \, [\log 1/I_A - 2*\log 1/I_B + \log 1/I_C] + K_2 T_S \tag{2}$$

wobei $K_1$ die Gefällinie von

$$[\log 1/I_A - 2*\log 1/I_B + 1/I_C]$$

ist, $\log 1/I_C$ einen Wert der optischen Dichte bei Wellenlänge C darstellt und C, $K_0$, $\log 1/I_A$ sowie $\log 1/I_B$ wie in Formel (1) definiert sind;

$$C = K_0 + K_1 \, \frac{[\log 1/I_A - \log 1/I_B]}{[\log 1/I_D - \log 1/I_E]} + K_2 T_S \tag{3}$$

wobei $K_1$ die Gefällinie von

$$\frac{[\log 1/I_A - \log 1/I_B]}{[\log 1/I_D - \log 1/I_E]}$$

ist, $\log 1/I_D$ und $\log 1/I_E$ jeweils einen Wert der optischen Dichte bei entsprechenden Wellenlängen D und E darstellen und C, $K_0$, $\log 1/I_A$ und $\log 1/I_B$ wie in Formel (1) definiert sind; oder

$$C = K_0 + K_1 \, \frac{[\log 1/I_A - 2*\log 1/I_B + \log 1/I_C]}{[\log 1/I_D - 2*\log 1/I_E + \log 1/I_F]} + K_2 T_S \tag{4}$$

wobei $K_1$ die Gefällinie von

$$\frac{[\log 1/I_A - 2*\log 1/I_B + \log 1/I_C]}{[\log 1/I_D - 2*\log 1/I_E + \log 1/I_F]}$$

ist, $\log 1/I_C$, $\log 1/I_D$, $\log 1/I_E$ und $\log 1/I_F$ jeweils einen Wert der optischen Dichte bei entsprechenden Wellenlängen C, D, E und F darstellen und C, $K_0$, $\log 1/I_A$ und $\log 1/I_B$ wie in Formel (1) definiert sind.

2. Analysegerät nach Anspruch 1, wobei die Energiequelle eine Vielzahl von Infrarot-C-emittierenden Dioden (16,16') umfaßt.

3. Analysegerät nach Anspruch 2, wobei die Vielzahl Infrarot-C-emittierender Dioden (16,16') sechs Infrarot-C-emittierende Dioden umfaßt.

4. Analysegerät nach einem der Anprüche 1 bis 3, wobei optische Filter (123), die den Optimalwellenlängenwerten für A und B für einen bestimmten Untersuchten entsprechen, in dem Gerät (10) installiert sind.

5. Nicht-invasives Verfahren für die quantitative Analyse von Blutglukose im Blut eines Untersuchten, das umfaßt:

a) Einleiten wenigstens eines Paars Wellenlängen mit Infrarot-C-Energie von einer Quelle (16,16') von Infrarot-C-Energie in Blut in einem Körperteil des Untersuchten, wobei das Paar Wellenlängen in dem Bereich von ungefähr 600 bis ungefähr 1100 Nanometer liegt;

b) Erfassen von Infrarot-C-Energie, die aus dem Untersuchten austritt, mit einem Detektor (28), der beim Erfassen von Energie, die aus dem Untersuchten austritt, ein erstes Signal erzeugt; und

c) Verarbeiten des ersten Signals, um ein zweites Signal zu erzeugen, das die Menge an Glukose anzeigt, die im Körper des Untersuchten vorhanden ist,

**dadurch gekennzeichnet**, daß
das erste Signal entsprechend einer Formel verarbeitet wird, die einen Temperaturterm $K_2 T_S$ enthält, wobei $T_S$ für die lokale Oberflächentemperatur des Körperteils steht und $K_2$ eine Eichkonstante ist, und wobei es sich bei der Formel um eine der folgenden handelt:

$$C = K_0 + K_1 [\log 1/I_A - \log 1/I_B] + K_2 T_S \qquad (1)$$

wobei C die Konzentration von Glukose ist, die in dem Blut vorhanden ist, $K_0$ eine Achsenabschnittskonstante ist, $K_1$ die Gefällinie von

$$[\log 1/I_A - \log 1/I_B]$$

ist und $\log 1/I_A$ und $\log 1/I_B$ jeweils einen Wert der optischen Dichte bei entsprechenden Wellenlängen A und B darstellen;

$$C = K_0 + K_1 [\log 1/I_A - 2{*}\log 1/I_B + \log 1/I_C] + K_2 T_S \qquad (2)$$

wobei $K_1$ die Gefällinie von

$$[\log 1/I_A - 2{*}\log 1/I_B + 1/I_C]$$

ist, $\log 1/I_C$ einen Wert der optischen Dichte bei Wellenlänge C darstellt und C, $K_0$ sowie $\log 1/I_A$ und $\log 1/I_B$ wie in Formel (1) definiert sind;

$$C = K_0 + K_1 \frac{[\log 1/I_A - \log 1/I_B]}{[\log 1/I_D - \log 1/I_E]} + K_2 T_S \qquad (3)$$

wobei $K_1$ die Gefällinie von

$$\frac{[\log 1/I_A - \log 1/I_B]}{[\log 1/I_D - \log 1/I_E]}$$

ist, $\log 1/I_D$ und $\log 1/I_E$ jeweils einen Wert der optischen Dichte bei entsprechenden Wellenlängen D und E darstellen und C, $K_0$, $\log 1/I_A$ und $\log 1/I_B$ wie in Formel (1) definiert sind;

$$C = K_0 + K_1 \frac{[\log 1/I_A - 2{*}\log 1/I_B + \log 1/I_C]}{[\log 1/I_D - 2{*}\log 1/I_E + \log 1/I_F]} + K_2 T_S \qquad (4)$$

wobei $K_1$ die Gefällinie von

$$\frac{[\log 1/I_A - 2*\log 1/I_B + \log 1</I_C]}{[\log 1/I_D - 2*\log 1/I_E + \log 1/I_F]}$$

ist, $\log 1/I_C$, $\log 1/I_D$, $\log 1/I_E$ und $\log 1/I_F$ jeweils einen Wert der optischen Dichte bei entsprechenden Wellenlängen C, D, E und F darstellen und C, $K_0$, $\log 1/I_A$ und $\log 1/I_B$ wie in Formel (1) definiert sind; oder

$$C = K_0 + K_1 \frac{[\log 1/I_A - \log 1/I_B]}{[\log 1/I_D - \log 1/I_E]} + K_2 T_S + K_3 T_A \qquad (5)$$

wobei $K_1$ die Gefällinie von

$$\frac{[\log 1/I_A - \log 1/I_B]}{[\log 1/I_D - \log 1/I_E]}$$

ist, $K_3$ eine Eichkonstante ist, $\log 1/I_D$ und $\log 1/I_E$ jeweils einen Wert der optischen Dichte bei entsprechenden Wellenlängen D und E darstellen, $T_A$ die Umgebungstemperatur des Gerätes darstellt und C, $K_0$, $\log 1/I_A$ und $\log 1/I_B$ wie in Formel (1) definiert sind.

6. Verfahren nach Anspruch 5, wobei das wenigstens eine Paar Wellenlängen auf folgende Weise individuell für einen Untersuchten ausgewählt wird:

a) aufeinanderfolgendes Einleiten von Infrarot-C-Energie in den Körperteil eines Untersuchten bei einer Vielzahl von Mittelwellenlängen, wobei die Vielzahl von Mittelwellenlängen durch eine konstante Wellenlänge getrennt sind;

b) Messen eines Glukosespiegels des Untersuchten bei einem niedrigen Glukosespiegel und Messen eines Glukosespiegels des Untersuchten bei einem höheren Glukosespiegel; und

c) Bestimmen, welche Mittelwellenlängen den größten Unterschied ergeben, anhand des Meßschritts und Verwendung der bestimmten Wellenlängen in anschließenden Messungen für den Untersuchten.

**Revendications**

1. Instrument (10) d'analyse quantitative dans le proche infrarouge permettant d'effectuer une mesure non-invasive de la teneur en glucose du sang présent dans le sang d'une partie du corps d'un patient, comprenant :

(a) des moyens d'introduction comprenant une source d'énergie dans le proche infrarouge (16, 16') pour introduire une énergie dans le proche infrarouge à l'intérieur du sang présent dans une partie du corps d'un patient;
(b) des moyens de détection (28) pour détecter l'énergie dans le proche infrarouge qui sort d'une partie du corps;
(c) des moyens de positionnement (20) pour positionner à la fois les moyens d'introduction (16, 16') dans le proche infrarouge et les moyens de détection (28) à proximité immédiate de la partie du corps ; et
(d) des moyens de traitement (34) pour traiter un premier signal électrique produit par les moyens de détection (28) et les transformer en un second signal indicateur de la quantité de glucose présent dans le sang du patient,

ledit instrument étant caractérisé en ce que les moyens de traitement traitent le premier signal en conformité avec une formule qui comprend un terme de température $K_2 T_S$ dans lequel $T_S$ représente la température de surface locale de ladite partie du corps et $K_2$ est une constante d'étalonnage et dans laquelle la formule est l'une des formules suivantes:

$$C = K_0 + K_1 [\log 1/I_A - \log 1/I_B] + K_2 T_S \qquad \text{(i)}$$

dans laquelle C est la concentration de glucose présent dans le sang, $K_0$ est une constante d'interception, $K_1$ est la pente de la droite de

$$[\log 1/I_A - \log 1/I_B],$$

et $\log 1/I_A$ et $\log 1/I_B$ représentent chacun une valeur de densité optique aux longueurs d'onde correspondantes A et B ;

$$C = K_0 + K_1 [\log 1/I_A - 2*\log 1/I_B + \log 1/I_C] + K_2 T_S \qquad \text{(ii)}$$

dans laquelle $K_1$ est la pente de la droite de

$$[\log 1/I_A - 2*\log 1/I_B + 1/I_C],$$

$\log 1/I_C$ représente une valeur de densité optique pour la longueur d'onde C, et C, $K_0$, $\log 1/I_A$ et $\log 1/I_B$ sont tels que définis dans la formule (i) ;

$$C = K_0 + K_1 \frac{[\log 1/I_A - \log 1/I_B]}{[\log 1/I_D - \log 1/I_E]} + K_2 T_S \qquad \text{(iii)}$$

dans laquelle $K_1$ est la pente de la droite de

$$\frac{[\log 1/I_A - \log 1/I_B]}{[\log 1/I_D - \log 1/I_E]}$$

$\log 1/I_D$ et $\log 1/I_E$ représentent chacun une valeur de densité optique aux longueurs d'onde correspondantes D et E, et C, $K_0$, $\log 1/I_A$ et $\log 1/I_B$ sont définis comme dans la formule (i) ; ou

$$C = K_0 + K_1 \frac{[\log 1/I_A - 2*\log 1/I_B + \log 1/I_C]}{[\log 1/I_D - 2*\log 1/I_E + \log 1/I_F]} + K_2 T_S \qquad \text{(iv)}$$

dans laquelle $K_1$ est la pente de la droite de

$$\frac{[\log 1/I_A - 2*\log 1/I_B + \log 1/I_C]}{[\log 1/I_D - 2*\log 1/I_E + \log 1/I_F]}$$

$\log 1/I_C$, $\log 1/I_D$, $\log 1/I_E$ et $\log I/I_F$ représentent chacun une valeur de densité optique aux longueurs d'onde correspondantes C, D, E et F, et C, $K_0$, $\log 1/I_A$ et $\log 1/I_B$ sont tels que définis dans la formule (i).

2. Instrument d'analyse selon la revendication 1 dans lequel ladite source d'énergie comprend une pluralité de diodes (16, 16') émettant dans le proche infrarouge.

3. Instrument d'analyse selon la revendication 2 dans lequel ladite pluralité de diodes émettant dans le proche infrarouge (16, 16') comprennent six diodes émettant dans le proche infrarouge.

4. Instrument d'analyse selon l'une quelconque des revendications 1 à 3, dans lequel des filtres optiques (123) correspondant aux valeurs optimales des longueurs d'onde pour A et B pour un patient donné sont installés dans ledit instrument (10).

5. Procédé non-invasif pour analyser la quantité du glucose du sang contenu dans le sang d'un patient, comprenant:

(a) l'introduction d'au moins une paire de longueurs d'onde d'énergie dans le proche infrarouge à partir d'une source d'énergie dans le proche infrarouge (16, 16') à l'intérieur du sang contenu dans une partie du corps du patient, ladite paire de longueurs d'onde étant située dans la plage d'environ 600 à environ 1100 nanomètres ;

(b) la détection de l'énergie dans le proche infrarouge qui sort du patient au moyen d'un détecteur (28) qui fournit un premier signal en détectant l'énergie qui sort du patient ; et

(c) le traitement du premier signal pour fournir un second signal indicateur de la quantité de glucose présente dans le corps du patient,

ledit procédé étant caractérisé en ce que le premier signal est traité en conformité avec une formule comprenant un terme de température $K_2 T_S$ dans lequel $T_S$ représente la température de surface locale de ladite partie du corps et $K_2$ est une constante d'étalonnage, et dans lequel procédé la formule est l'une des suivantes

$$C = K_0 + K_1 [\log 1/I_A - \log 1/I_B] + K_2 T_S \qquad (i)$$

dans laquelle C est la concentration de glucose présent dans le sang, $K_0$ est une constante d'interception, $K_1$ est la pente de la droite de

$$[\log 1/I_A - \log 1/I_B],$$

$\log 1/I_A$ et $\log 1/I_B$ représentent chacun une valeur de densité optique aux longueurs d'onde correspondantes A et B ;

$$C = K_0 + K_1 [\log 1/I_A - 2*\log 1/I_B + \log 1/I_C] + K_2 T_S \qquad (ii)$$

dans laquelle $K_1$ est la pente de la droite de

$$[\log 1/I_A - 2*\log 1/I_B + I/I_C],$$

$\log 1/I_C$ représente une valeur de densité optique pour la longueur d'onde C, et C, $K_0$, $\log 1/I_A$ et $\log 1/I_B$ sont tels que définis dans la formule (i);

$$C = K_0 + K_1 \frac{[\log 1/I_A - \log 1/I_B]}{[\log 1/I_D - \log 1/I_E]} + K_2 T_S \qquad (iii)$$

dans laquelle $K_1$ est la pente de la droite de

$$\frac{[\log 1/I_A - \log 1/I_B]}{[\log 1/I_D - \log 1/I_E]}$$

$\log 1/I_D$ et $\log 1/I_E$ représentent chacun une valeur de densité optique aux longueurs d'onde correspondantes D et E, et C, $K_0$, $\log 1/I_A$ et $\log 1/I_B$ sont définis comme dans la formule (i) ;

$$C = K_0 + K_1 \frac{[\log 1/I_A - 2*\log 1/I_B + \log 1/I_C]}{[\log 1/I_D - 2*\log 1/I_E + \log 1/I_F]} + K_2 T_S \qquad (iv)$$

dans laquelle $K_1$ est la pente de la droite de

$$\frac{[\log 1/I_A - 2*\log 1/I_B + \log 1/I_C]}{[\log 1/I_D - 2*\log 1/I_E + \log 1/I_F]}$$

et $\log 1/I_C$, $\log 1/I_D$, $\log 1/I_E$, $\log 1/I_F$ représentent chacun une valeur de densité optique aux longueurs d'onde correspondantes C, D, E et F, et C, $K_0$, $\log 1/I_A$ et $\log 1/I_B$ sont tels que définis dans la formule (i); ou

$$C = K_0 + K_1 \frac{[\log 1/I_A - \log 1/I_B]}{[\log 1/I_D - \log 1/I_E]} + K_2 T_S + K_3 T_A \qquad \text{(v)}$$

dans laquelle $K_1$ est la pente de la droite de

$$\frac{[\log 1/I_A - \log 1/I_B]}{[\log 1/I_D - \log 1/I_E]}$$

$K_3$ est une constante d'étalonnage, $\log 1/I_D$ et $\log 1/I_E$ représentent chacun une valeur de densité optique aux longueurs d'onde correspondantes D et E, $T_A$ représente la température ambiante dudit instrument, et C, $K_0$, $\log 1/I_A$ et $\log 1/I_B$ sont tels que définis dans la formule (i).

**6.** Procédé selon la revendication 5, dans lequel ladite au moins une paire de longueurs d'onde est sélectionnée individuellement pour un patient de la façon suivante:

(a) on introduit séquentiellement une énergie dans le proche infrarouge à l'intérieur de ladite partie du corps d'un patient sous une pluralité de centres de longueurs d'onde, ladite pluralité de centres de longueurs d'ondes étant séparée par une longueur d'onde constante ;

(b) on mesure le niveau de glucose dudit patient à un moment de bas niveau de glucose et on mesure le niveau de glucose dudit patient à un moment de plus haut niveau de glucose; et

(c) on determine à partir de l'étape de mesure lequel centre de longueurs d'onde fournit la différence maximale et l'on utilise les longueurs d'onde déterminées dans les mesures subséquentes pour ce patient.

# FIG. 1

# FIG. 3

# FIG. 2A

# FIG. 2B

# FIG. 4

# FIG. 5A

17 — LIGHT SOURCE

— LENS

— WIDEBAND LIGHT BEAM

23' — TILTING FILTER
WAVELENGTH VARIES AS
FILTER TURNS

— NARROWBAND LIGHT BEAM

— OPTICAL DETECTOR

# FIG. 5B

LIGHT SOURCE
17'

23" — DISK WITH
OPTICAL FILTERS

— OPTICAL DETECTOR

## FIG. 6

# FIG. 7

EP 0 536 304 B1

FIG. 8

FIG. 9

# FIG. 10

# FIG. II

EP 0 536 304 B1

FIG. 12

PROTEIN

WATER

REGION "K"

REGION "J"

FAT

WAVELENGTH UM

EP 0 536 304 B1

FIG. 13

EP 0 536 304 B1

# FIG. 14

ABSORPTION BAND (0.9-1.05μ) OF LIQUID WATER AT 0.5° TO 90°C.

# FIG. 15

# FIG. 16

FIG. 17

EP 0 536 304 B1

# FIG. 18A

CLASS 1

CORRELATION

+1.0

0

WAVELENGTH

>6nm

# FIG. 18B

CLASS 2

CORRELATION

+1.0

0

WAVELENTH

~4-6nm

# FIG. 18C

CLASS 3

CORRELATION

+1.0

0

WAVELENGTH

~2-3nm

# FIG. 18D

CLASS 4

CORRELATION

+1.0

0

WAVELENGTH

<2nm

# FIG. 19

EP 0 536 304 B1

# FIG. 20

# FIG. 21

# FIG. 22

1

196

# FIG. 23

198

195

197B

RAM

197A

EPROM

BATTERY
POWER

189

184

# FIG. 24A

OFFICIAL
LABORATORY
GLUCOSE
VALUE
mg/dl

400

300

200

100

PERFECT
CORRELATION
LINE

TYPICAL
CALIBRATION
RANGE

100    200    300    400    mg/dl

NEAR-IR INSTRUMENT
GLUCOSE VALUE

# FIG. 24B

OFFICIAL
LABORATORY
GLUCOSE
VALUE
mg/dL

400

300

200

100

PERFECT
CORRELATION
LINE

POTENTIAL
ERROR BAND
IF "POPULATION"
MATH TERMS
ARE USED

TYPICAL
CALIBRATION
RANGE

100    200    300    400    mg/dl

NEAR-IR INSTRUMENT
GLUCOSE VALUE

# FIG. 24C

OFFICIAL LABORATORY GLUCOSE VALUE mg/dl

PERFECT CORRELATION LINE

POTENTIAL ERROR IF SINGLE TERM NORMALIZED MATH IS USED

TYPICAL CALIBRATION RANGE

NEAR-IR INSTRUMENT GLUCOSE VALUE

# FIG. 24D

OFFICIAL LABORATORY GLUCOSE VALUE mg/dl

PERFECT CORRELATION LINE

POTENTIAL ERROR IF 2 TERM NORMALIZED MATH IN USE

TYPICIAL CALIBRATION RANGE

NEAR-IR INSTRUMENT GLUCOSE VALUE

FIG. 25A    31A    31B    FIG. 25B

FIG. 26

266    250

RAM    278A

EPROM    278B

258    BATTERY
POWER    276

256    279    280

252    264

ELECTRONICS
&
PROCESSOR

262

274    263

254    260    268    270

# FIG. 27

266

272

250

# FIG. 28

OBTAIN BLOOD SAMPLES AT PREDETERMINED TIME INTERVAL AND FOR PREDETERMINED PERIOD OF TIME

OBTAIN BLOOD GLUCOSE LEVEL MEASUREMENTS OF SAMPLES AND ENTER INTO ANALYSIS INSTRUMENT

SIMULTANEOUSLY OBTAIN NEAR-INFRARED OPTICAL MEASUREMENTS THROUGH THE INDIVIDUAL'S BODY PART

LINEARLY INTERPOLATE SAMPLE GLUCOSE MEASUREMENTS WITH THE NEAR-INFRARED OPTICAL MEASUREMENTS TO CUSTOM CALIBRATE THE INSTRUMENT

# FIG. 29

LABORATORY
GLUCOSE
METER

TYPE IN

311

PORTABLE
GLUCOSE
METER

OPTICAL DATA

330

2nd
REPLACEMENT
CARTRIDGE

CALIBRATION
CONSTANTS

CALIBRATION
SYSTEM

320

KEYPAD

320

FIRST
REPLACEABLE
CARTRIDGE

332

PRINT OUT

# FIG. 30

LABORATORY
GLUCOSE
METER

TYPE IN

KEYPAD
442

441

PORTABLE
GLUCOSE
METER

DOCTOR
OFFICE
UNIT

420

FIRST
REPLACEABLE
CARTRIDGE

SECOND
REPLACEMENT
CARTRIDGE

PRINT OUT

INSERT
REPLACEMENT
CARTRIDGE

# FIG. 31

INFLUENCE OF TEMPERATURE IN
THE VERY-NEAR-IR